# EUROPEAN PATENT SPECIFICATION

(11) **EP 3 250 902 B1**
(45) Date of publication and mention of the grant of the patent: **08.05.2024**
(21) Application number: 16702361.3
(22) Date of filing: 25.01.2016
(51) Int. Cl.: G01N 1/30, C12Q 1/6886, G01N 33/574

(54) **METHODS FOR TISSUE SAMPLE FIXATION USING AN EXTENDED SOAK IN ALDEHYDE-BASED FIXATIVE SOLUTIONS**
VERFAHREN ZUM FIXIEREN VON GEWEBEPROBEN DURCH LANGES EINTAUCHEN IN ALDEHYD BASIERTEN FIXATIVEN
PROCÉDÉ DE FIXATION D'ÉCHANTILLONS DE TISSUES PAR TREMPAGE PROLONGÉ DANS UNE SOLUTION DE FIXATEUR À BASE D'UN ALDÉHYDE

(30) Priority: 27.01.2015 US 201562108248 P
(43) Date of publication of application: 06.12.2017
(73) Proprietor: Ventana Medical Systems, Inc., Tucson, Arizona 85755 (US)
(72) Inventor: CHAFIN, David, Tucson, Arizona 85755 (US); OTTER, Michael, Tucson, Arizona 85755 (US)
(74) Representative: Simmons & Simmons LLP (Munich)
(86) International application number: PCT/EP2016/051431
(87) International publication number: WO 2016/120195

(56) References cited:
- WO-A1-02/23156
- WO-A1-02/23156
- WO-A1-87/00004
- WO-A1-2014/100079
- WO-A1-2014/100079
- WO-A2-2008/024837
- WO-A2-2008/024837
- US-A- 4 656 047
- US-A1- 2011 053 171
- US-A1- 2011 053 171
- US-A1- 2012 214 195

## Description

### BACKGROUND OF THE DISCLOSURE

### I. Field of the Invention

The present application relates to fixation methods for preserving tissue samples.

### II. Brief Description of Related Art

Proper medical diagnosis and patient safety require properly fixing prior to staining. The most common method of fixation for clinical diagnostic purposes is to immerse the tissue sample in 10% neutral buffered formalin (NBF) at room temperature. Unfortunately, many downstream analytical methods are highly sensitive to the amount of time spent in NBF. For example, if a tissues that have been exposed to formalin for a substantially extended period of time often do not work well for subsequent histochemical processes. The widely expressed cancer marker protein p53, for example, gradually loses all of its reactivity toward monoclonal antibody PAb1801 when fixed in formaldehyde for between 6 and 24 hours. Silvestrini et al., 87 J. Nat. Cancer Inst. 1020 (1995). Similarly, the diagnostically important epithelial cell marker protein keratin gradually becomes unable to bind with a monoclonal anti-keratin antibody if the tissue is fixed in formaldehyde for up to 24 hours. Battifora & Kopinski, 34 J. Histochem. Cytochem. 1095-1100 (1986). Other antibodies are sensitive to fixation time in room temperature NBF, including, for example, lymphocyte antigens, vimentin, desmin, neurofilaments, cytokeratins, S100 protein, prostate specific antigen, thyroglobulin, and carcinoembryonic antigen. Leong & Gilham, 4 Pathology 266-268 (1989). Similarly, nucleic acid analyses are often sensitive to fixation time. *See* Srinivasan, Am J Pathol., vol. 161, issue 6, p. 1961-71 (2002); O'Leary et al., 26 Histochem. J. 337-346 (1994); Greer et al., 95 Am. J. Clin. Pathol. 117-124 (1991); F. Karisen et al., 71 Lab. Invest. 604-611(1994). Others have shown that post-translational modifications to some proteins, such as phosphorylation, are sensitive to extended room temperature NBF exposure. *See* Mueller et al., PLoS One, Vol. 6(8): e23780 (2011).

Thus, under current clinical practice, it is important to control the tissue fixation time to achieve a compromise between the preservation of tissue morphology and the loss of antigenicity. For example, ASCO guidelines suggest fixation of tissues for at least 6 hours but no more than 72 hours if the sample is to be assayed for HER2 expression immunohistochemically. However, it often is not practical to minimize the extent of exposure to NBF. For example, tissue sample collected toward the end of the week may often be stored at room temperature in fixative over a weekend before they can be further processed. In other cases, the tissue sample may be collected at one site and then transported to a second site for further processing, which can add to processing times. In each of these cases, it is not uncommon for the amount of time in room temperature NBF. Indeed, Leong and Gilham report that the bulk of a typical surgical resection is often retained in NBF for future resampling, which may occur after 3 or more days. Similarly, autopsy specimens are usually fixed for between 3 and 14 days, depending on convenience of the technician. As a result, the quality of fixation for tissue samples is inconsistent, which can lead to variable results in downstream analytical methods and even missed diagnoses.

Some methods have been developed to address these problems.

For example, it is known to use fast freezing methods in order to halt the action of modification enzymes. *See* Lawson et. al. Cryobiology, vol. 62, issue 2, 115-22 (2011). Although fast freezing may initially slow down the action of such enzymes, it does not completely inhibit their action upon thawing of the sample and thus does not always ameliorate loss of labile biomarkers. Additionally, fast freezing methods are not commonly used in commercial histology laboratories, and thus would require adoption of completely different reagents and systems.

US Patent 8,460,859 B2 discloses the use of a three-part special fixative to achieve the stabilization of phosphoproteins. The fixative comprises a preservation component, a stabilizer component and a permeability enhancing component. In order to obtain long term preservation, the patent requires that the tissue sample be frozen. However, these methods are more complicated than can practically be applied on a commercial scale.

Others have tried to mitigate the effect of endogenous degradation pathways by fixing the tissues in the presence of exogenous protease and nuclease inhibitors to prevent loss of potential analytes during fixation. *See* WO 2011-130280 A1 and WO 2008-073187 A2. However, direct inhibition of naturally occurring pathways in the tissue can affect the end results. For example, WO 2008-073187 A2 teaches that treatment of tissues with phosphatase inhibitors can cause "highly abnormal upward accumulation of abnormal levels of phosphoproteins." These methods thus do not yield reliable results. Moreover, the amounts of inhibitors necessary to adequately block enzyme activity makes the methods cost-prohibitive to implement on a wide scale.

The present inventors are not aware of any existing methods to sufficiently mitigate negative effects of extended exposure of tissue samples to fixative solutions without resorting to special reagents or complicated processing steps.

### SUMMARY

The present invention is directed to improved methods for preserving biomarkers when a tissue sample is subjected to aldehyde fixation. The aldehyde-based fixative solution and tissue sample are typically in contact with each other at the first temperature range for a period of time effective to allow the aldehyde-based fixative solution to diffuse throughout substantially the entire cross section of the tissue sample without significant diffusion inhibiting cross-linking occurring for up to 14 days. After exposure to fixative at the first temperature or temperature range the tissue sample is exposed to a second higher temperature for a second period of time sufficient to induce cross-linking. The methods enable post-fixation processing of tissue samples to be delayed up to 14 days and perhaps longer while maintaining excellent preservation of tissue morphology, antibody reactivity, and labile biomarkers.

Embodiments of the method comprise applying a first aldehyde-based fixative solution at a first temperature to a tissue sample, followed by applying a second aldehyde-based fixative solution to the tissue sample. In some embodiments of the present invention, a first temperature range is from at least 0°C to about 10°C. In at least one embodiment the temperature can be in the range from about 2°C to about 8°C, while in another embodiment can be in the range from about 4°C, plus or minus 3 °C. Embodiments of the invention may have a time range during which the tissue sample is exposed to the aldehyde-based fixative solution at the first temperature of from about 72 hours up to about 14 days or more.

The second aldehyde-based fixative solution may be different from the first aldehyde-based fixative solution. For example, the solutions can be at different concentrations, or the second aldehyde-based fixative solution may comprise an aldehyde different from the first aldehyde. The aldehyde typically is a lower alkyl aldehyde, such as formaldehyde, glyoxal, glutaraldehyde, or combinations thereof.

The method of the present invention is defined in claim 1.

One disclosed exemplary embodiment of the present invention comprises immersing a tissue sample into a formalin solution at a temperature of from equal to or greater than 0 °C up to 7 °C for a first period of from greater than 72 hours up to about 14 days. The tissue sample is then immersed into a formalin solution at a second temperature greater than about 20°C up to at least 45°C for a second time period of from about 1 hour to about 4 hours. The formalin solution generally is 10% - 30% NBF. These processing steps typically are followed by a series of alcohol washes, further followed by a clearing solution wash, such as a xylene wash, of from greater than 0 minutes up to at least about 30 minutes, or to about 1, about 2, about 3, or about 4 hours. Wax is then applied to the tissue sample to form a wax impregnated block.

Without being bound by a theory of operation, it currently is believed that at reduced temperature, very little cross-linking occurs but fixative solution does penetrate into substantially the whole tissue section. Additionally, it may be that metabolic or enzymatic processes are dramatically reduced. Once diffused, the temperature is rapidly raised, where cross-linking kinetics are greatly increased. In addition, since fixative solution has substantially diffused into the sample, more even morphologic and antigen preservation are observed. This protocol differs from the prior art by separating the fixation process into a first process step that permits diffusion of fixative solution into a tissue sample but minimizes cross-linking, and a second process step that increases the rate of cross-linking, during the time periods typically used for fixing a tissue sample in disclosed working embodiments.

In typical embodiments, the methods preserve post-translation modification signals of proteins in the tissue sample significantly, for example, by preserving at least 30%, 50%, 70%, or 90% post-translation modification signals for up to 14 days. The tissue fixation methods of the present invention can significantly halt the enzyme activities destroying the post-translation modification signals, such as halting the enzyme activities of phosphatase.

In another typical embodiment, the methods preserve signals of proteins in the tissue sample significantly, for example, by preserving at least 30%, 50%, 70%, or 90% post-translation modification signals. The tissue fixation methods of the present invention can significantly halt the enzyme activities degrading proteins, such as halting the enzyme activities of protease for up to 14 days.

In one exemplary embodiment, formaldehyde fixed-paraffin embedded (FFPE) tissue samples are used. The present method offers several advantages over existing attempts to preserve modification states from FFPE tissue. The method uses a standard formalin solution that is in wide use in histology practice. The cold step can be carried out in a simple manner consisting of cold formalin for up to 14 days followed by heated formalin. The present invention for the first time in the art accomplishes long term preservation of modification states in FFPE tissue.

In summary, the present method offers at least three improvements over existing methods in the art. First, by allowing formalin to penetrate into the tissue section in a cold environment can significantly reduce enzyme activities for up to 14 days. Second, by increasing the cross-linking kinetics by quickly raising the tissue sample temperature, the cellular constituents and biomarkers are "locked" into place more rapidly than what would be observed at room temperature. This combination makes this technique superior over existing methods and for the first time allows modification states to be preserved in FFPE tissues. Third, this represents a general method believed to be applicable to a wide variety of modification states and enzymes. While other methods target a specific set of modification enzymes, this method rapidly disables all modification enzymes and therefore preserve the general cellular status much better than gold standard room temperature procedures. Since the invention is not limited to a specific set of biomolecules or biomolecules containing specific post-translations modifications, it is believed that this method represents a general method for preservation of any biomolecule or modification state. Thus, this invention can preserve with high quality quantities of biomolecules and biomolecules containing specific post-translations modifications.

The foregoing and other objects, features, and advantages of the invention will become more apparent from the following detailed description, which proceeds with reference to the accompanying figures.

### BRIEF DESCRIPTION OF THE DRAWINGS

FIG. 1 illustrates 4mm Calu3 xeongraft tumor cores that were placed into cooled formalin at 7 °C, 10 °C or 15 °C for 2, 4 or 6 hours. 24 hour room temperature fixation and 2+2 (i.e. 2 hours at 4 °C followed by 2 hours at 45 °C) controls are also illustrated.
FIG. 2 are digital microscope images of 4mm Calu3 Xeongraft tumor cores that were placed into cooled formalin at 4 °C for 2 hours (Column A), 1 day (Column B), 2 days (Column C), 5 days (Column D), 7 days (Column E) and 14 days (Column F), followed by two hours in formalin at 45 °C.
FIGS. 3A and 3B are temperature profiles of shipping package 1 from Example 3.
FIGS. 4A and 4B are temperature profiles of shipping package 2 from Example 3.

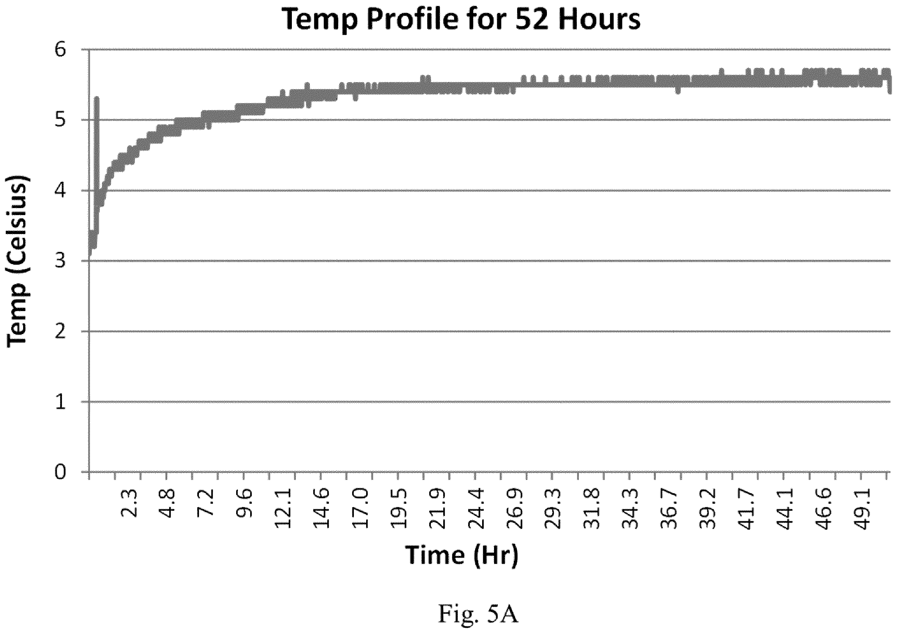

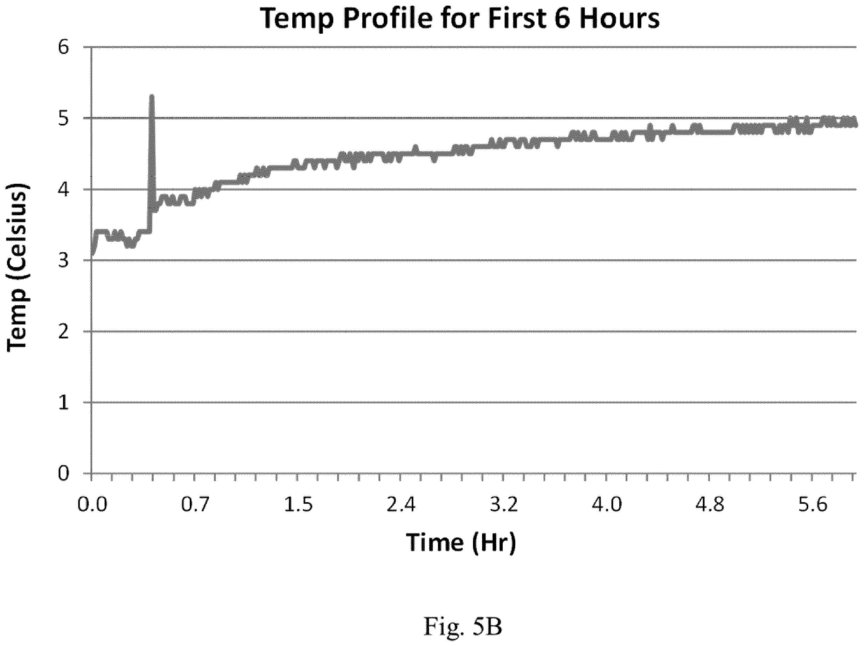

FIGS. 5A and 5B are temperature profiles of shipping package 3 from Example 3.

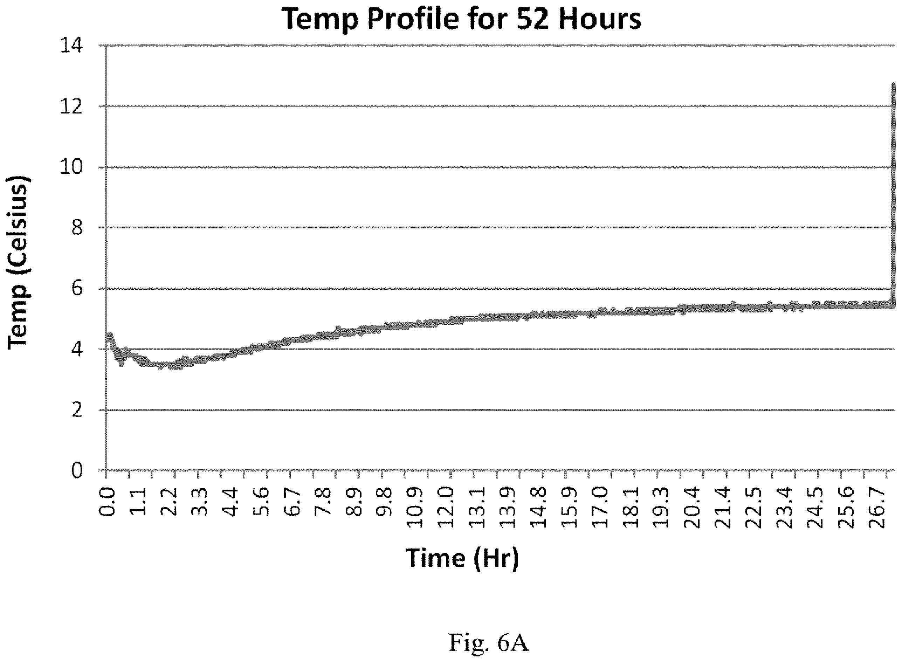

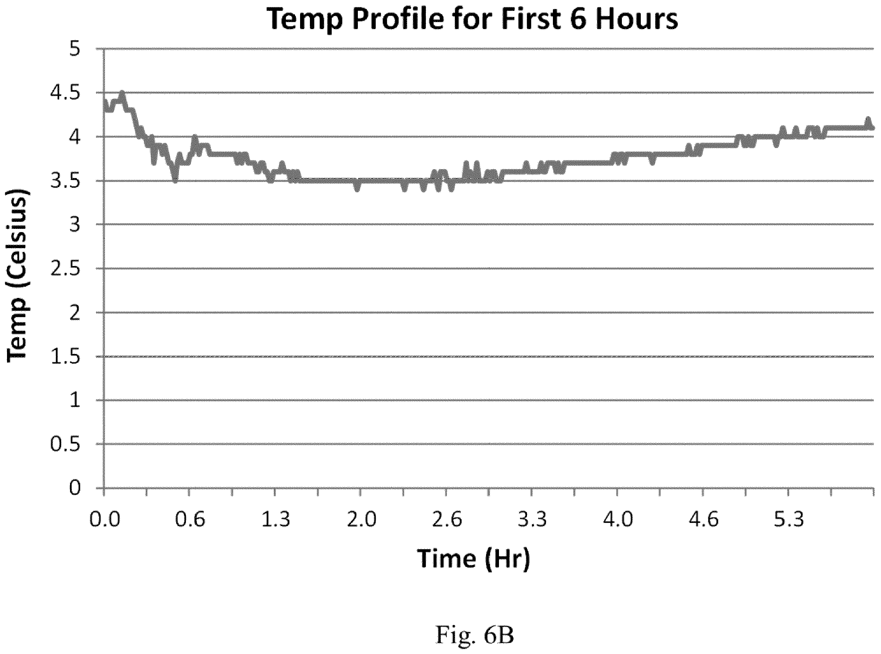

FIGS. 6A and 6B are temperature profiles of shipping package 4 from Example 3.
FIG. 7 illustrates digital microscope images of tonsil tissue stained with hematoxylin and eosin (H&E), or immunohistochemically stained for PD-L1, FoxP3, and CD68 expression. Tissues sections were either shipped according to Example 3 (row S) or fixed using the 2+2 process (row C). Column A corresponds to tissues used in shipment 1. Column B corresponds to tissues used in shipment 2. Column C corresponds to tissues used in shipment 3. Column D corresponds to tissues used in shipment 4.
Fig. 8 illustrates digital images of tonsil samples immunohistochemically stained for FoxP3 and heat maps showing the density of FoxP3 cells per mm². Row A are samples fixed for 24 hours in room temperature formalin. Row B are samples fixed using an extended cold soak (4 days at ~5 °C, followed by 1 hour at 45 °C). Row C are samples fixed for 2 hours in 4 °C formalin and then for 2 hours in 45 °C formalin.
Fig. 9 is a bar graph illustrating the density of FoxP3 cells per mm². 126-130 indicate separate replicates. For each replicate, the bars represent samples subjected to (from left to right): (1) 2+2 fixation; (2) an extended soak (4 days at ~5 °C, followed by 1 hour at 45 °C); and (3) 24 hours in room temperature formalin.
FIG. 10 illustrates digital microscope images of Calu-3 xenografts immunohistochemically stained for PR, Ki-67 and the phosphorylated AKT protein (pAKT). Tissue sections were either shipped according to Example 3 (row S) or fixed using the 2+2 process (row C). Column A corresponds to tissues used in shipment 1. Column B corresponds to tissues used in shipment 2. Column C corresponds to tissues used in shipment 3. Column D corresponds to tissues used in shipment 4.
Fig. 11 is a bar graph illustrating differences in p-AKT preservation between using a 24 hour room temperature and the shipping conditions outlined in Example 3.
Figs. 12A-12K illustrate pAkt preservation using a variety of cold/hot fixation conditions as set forth in Table 3. Images correspond to conditions as follows: 12B is Experiment 1.1; 12C is Experiment 1.2; 12D is Experiment 2.1; 12E is Experiment 2.2; 12F is experiment 2.3; 12G is experiment 2.4; 12H is experiment 3.1; 121 is experiment 4.1; 12J is experiment 5.1; 12K is experiment 6.1.
Fig. 13 is a digital image of tissue samples labeled for miR-21 or miR-200c by *in situ* hybridization after fixation at 24 hours in room temperature NBF (left column) or fixation in 4 °C NBF for 2 hours followed by 45 °C NBF for 2 hours (right column).

### DETAILED DESCRIPTION

### I. Abbreviations and Definitions

In order to facilitate review of the various examples of this disclosure, the following explanations of abbreviations and specific terms are provided:
**H&E:** Hematoxylin and eosin staining.
**FFPE tissue:** Formalin-fixed, paraffin-embedded tissue.
**IHC:** Immunohistochemistry.
**ISH:** *In situ* hybridization.
**NBF:** neutral buffered formalin.
**Affinity histochemistry:** A histochemical method in which the analyte-binding entity is an agent other than an antibody, antibody fragment, or nucleic acid probe.
**Aldehyde-based fixative:** Any composition suitable for fixation of a tissue sample in which at least one of the agents primarily responsible for tissue fixation is an aldehyde.
**Analyte:** An entity (such as a molecule, group of molecules, macromolecule, subcellular structure, or cell) that is to be specifically detected in a sample.
**Analyte-binding entity:** Any compound or composition that is capable of specifically binding to an analyte. Examples of analyte-binding entities include: antibodies and antibody fragments (including single chain antibodies), which bind to target antigens; t-cell receptors (including single chain receptors), which bind to MHQantigen complexes; MHC: peptide multimers (which bind to specific T-cell receptors); aptamers, which bind to specific nucleic acid or peptide targets; zinc fingers, which bind to specific nucleic acids, peptides, and other molecules; receptor complexes (including single chain receptors and chimeric receptors), which bind to receptor ligands; receptor ligands, which bind to receptor complexes; nucleic acid probes, which hybridize to specific nucleic acids; and engineered specific binding structures, including ADNECTINs (scaffold based on 10th FN3 fibronectin; Bristol-Myers-Squibb Co.), AFFIBODYs (scaffold based on Z domain of protein A from S. aureus; Affibody AB, Solna, Sweden), AVIMERs (scaffold based on domain A/LDL receptor; Amgen, Thousand Oaks, CA), dAbs (scaffold based on VH or VL antibody domain; GlaxoSmithKline PLC, Cambridge, UK), DARPins (scaffold based on Ankyrin repeat proteins; Molecular Partners AG, Zürich, CH), ANTICALINs (scaffold based on lipocalins; Pieris AG, Freising, DE), NANOBODYs (scaffold based on VHH (camelid Ig); Ablynx N/V, Ghent, BE), TRANS-BODYs (scaffold based on Transferrin; Pfizer Inc., New York, NY), SMIPs (Emergent Biosolutions, Inc., Rockville, MD), and TETRANECTINs (scaffold based on C-type lectin domain (CTLD), tetranectin; Borean Pharma A/S, Aarhus, DK). Descriptions engineered specific binding structures are reviewed by Wurch et al., Development of Novel Protein Scaffolds as Alternatives to Whole Antibodies for Imaging and Therapy: Status on Discovery Research and Clinical Validation, Current Pharmaceutical Biotechnology, Vol. 9, pp. 502-509 (2008).
**Antibody:** The term "antibody" herein is used in the broadest sense and encompasses various antibody structures, including but not limited to monoclonal antibodies, polyclonal antibodies, multispecific antibodies (e.g., bispecific antibodies), and antibody fragments so long as they exhibit the desired antigen-binding activity.
**Antibody fragment:** A molecule other than an intact antibody that comprises a portion of an intact antibody that binds the antigen to which the intact antibody binds. Examples of antibody fragments include but are not limited to Fv, Fab, Fab', Fab'-SH, F(ab')2; diabodies; linear antibodies; single-chain antibody molecules (e.g. scFv); and multispecific antibodies formed from antibody fragments.
**Anti-phospho-antibody:** An antibody or antibody fragment that binds to a phosphorylated protein or amino acid residue, but not to a non-phosphorylated version of the same protein or amino acid residue. Examples of anti-phospho antibodies include:
   - antibodies specific for a specific phosphorylated amino acid residue, such as phosphorylated histidine (anti-phospho-His), phosphorylated serine (anti-phospho-Ser), phosphorylated threonine (anti-phospho-Thr), and phosphorylated tyrosine (anti-phospho-Tyr); and
   - antibodies specific for a particular antigen containing a phosphorylated amino acid, e.g. Akt phosphorylated at serine 473 (anti-phospho-Akt (Ser473)).
**Antigen:** A compound, composition, or substance that may be specifically bound by the products of specific humoral or cellular immunity, such as an antibody molecule or T-cell receptor. Antigens can be any type of molecule including, for example, haptens, simple intermediary metabolites, sugars (e.g., oligosaccharides), lipids, and hormones as well as macromolecules such as complex carbohydrates (e.g., polysaccharides), phospholipids, nucleic acids and proteins. Common categories of antigens include, but are not limited to, viral antigens, bacterial antigens, fungal antigens, protozoa and other parasitic antigens, tumor antigens, antigens involved in autoimmune disease, allergy and graft rejection, toxins, and other miscellaneous antigens.
**Cellular sample:** A sample comprising a collection of cells obtained from a subject or patient. Examples of cellular samples herein include, but are not limited to, tumor biopsies, circulating tumor cells, serum or plasma, primary cell cultures or cell lines derived from tumors or exhibiting tumor-like properties, as well as preserved tumor samples, such as formalin-fixed, paraffin-embedded tumor samples or frozen tumor samples.
**Clinical cellular sample:** A cellular sample obtained directly from a human or veterinary subject for the purpose of diagnosing a disease or disorder, determining a prognosis of a disease or disorder, and/or predicting response of a disease or disorder to a particular course of treatment.
**Clinical sample:** A sample obtained directly from a human or veterinary subject for the purpose of diagnosing a disease or disorder, determining a prognosis of a disease or disorder, and/or predicting a response of a disease or disorder to a particular course of treatment.
**Clinical tissue sample:** A tissue sample obtained directly from a human or veterinary subject for the purpose of diagnosing a disease or disorder, determining a prognosis of a disease or disorder, and/or predicting response of a disease or disorder to a particular course of treatment.
**Formalin:** A saturated aqueous solution of formaldehyde, which typically contains ~40% formaldehyde by volume (~37% by mass). Also referred to as "100% formalin." In aqueous solution, formaldehyde forms a hydrate, methanediol (H₂C(OH)₂), which exists in equilibrium with various formaldehyde oligomers, depending on the concentration and temperature. Therefore, a small amount of stabilizer, such as methanol, is usually added to suppress oxidation and polymerization. A typical commercial grade formalin may contain 10-15% methanol in addition to various metallic impurities.
**Histochemistry:** A method of evaluating a tissue sample by contacting the sample with an analyte-binding entity in a manner that causes a detectable marker (such as a dye, chromogen, or a fluorophore) to deposited on the sample in close proximity to the analyte. Examples of histochemistry include primary staining (such as H&E stains, acid-fast bacterial stains, etc.), immunohistochemistry, *in situ* hybridization, and affinity histochemistry.
**Immunohistochemistry:** A histochemical method in which the analyte-binding entity comprises an antibody or antibody fragment.
***In situ* hybridization:** A histochemical method in which the analyte is a nucleic acid and the analyte-binding entity comprises a nucleic acid probe complementary to the analyte nucleic acid.
**Kinase:** Any polypeptide - or complex or fragment thereof - that catalyzes the formation of a phosphate bond on a biomolecule.
**Kinase inhibitor:** Any molecule that specifically inhibits the ability of a kinase to catalyze the formation of a phosphate bond.
**Nuclease:** Any polypeptide - or complex or fragment thereof - that catalyzes the cleavage of the phosphodiester bonds between the nucleotide subunits of nucleic acids.
**Nuclease inhibitor:** Any molecule that specifically inhibits the ability of a nuclease to catalyze the cleavage of the phosphodiester bonds between the nucleotide subunits of nucleic acids.
**Oligopeptide:** A peptide from 2 to 20 amino acids in length.
**Peptide:** The term "peptide" is intended to encompass any arrangement of two or more amino acids joined together by amide bonds, including oligopeptides and polypeptides. When the amino acids are alpha-amino acids, either the L-optical isomer or the D-optical isomer can be used.
**Phosphatase:** Any polypeptide - or complex or catalytically-active fragment thereof - that catalyzes the cleavage of a phosphate bond.
**Phosphatase inhibitor:** Any molecule that specifically inhibits the ability of a phosphatase to cleave a phosphate bond.
**Protease:** Any polypeptide - or complex or fragment thereof - that catalyzes the cleavage of a peptide bond.
**Protease inhibitor:** Any molecule that specifically inhibits the ability of a protease to catalyze the cleavage of a peptide bond.
**Polypeptide:** A peptide longer than 20 amino acids in length. The terms "polypeptide" or "protein" as used herein are intended to encompass any amino acid sequence and include modified sequences such as glycoproteins.
**Post-translation modification:** A chemical modification of a protein after its translation. It is one of the later steps in protein biosynthesis, and thus gene expression, for many proteins. The post-translational modification of amino acids extends the range of functions of the protein by attaching it to other biochemical functional groups (such as acetate, phosphate, various lipids and carbohydrates), changing the chemical nature of an amino acid (e.g. citrullination), or making structural changes (e.g. formation of disulfide bridges). Also, enzymes may remove amino acids from the amino end of the protein, or cut the peptide chain in the middle. For instance, the peptide hormone insulin is cut twice after disulfide bonds are formed, and a pro-peptide is removed from the middle of the chain; the resulting protein consists of two polypeptide chains connected by disulfide bonds. Also, most nascent polypeptides start with the amino acid methionine because the "start" codon on mRNA also codes for this amino acid. This amino acid is usually taken off during post-translational modification. Other modifications, like phosphorylation, are part of common mechanisms for controlling the behavior of a protein, for instance activating or inactivating an enzyme.
**Sample:** A biological specimen obtained from a subject or patient containing genomic DNA, RNA (including mRNA), protein, or combinations thereof. Examples include, but are not limited to, peripheral blood, urine, saliva, tissue biopsy, surgical specimen, amniocentesis samples and autopsy material.
**Specific binding:** Specific binding occurs when an entity binds to an analyte in a sample to the substantial exclusion of binding to other potential analytes. For example, an entity may be considered to specifically bind to a given molecule when it has a binding constant that is at least 10³ M⁻¹ greater, 10⁴ M⁻¹ greater or 10⁵ M⁻¹ greater than a binding constant for other molecules in the sample.
**Tissue sample:** A cellular sample that preserves the cross-sectional spatial relationship between the cells as they existed within the subject from which the sample was obtained. "Tissue sample" shall encompass both primary tissue samples (i.e. cells and tissues produced by the subject) and xenografts (i.e. foreign cellular samples implanted into a subject).
**"X-% formalin":** A liquid composition containing an equivalent amount of formaldehyde as formalin (as defined above) diluted in a solvent to the specified percentage on a volume to volume basis. Thus, for example, a 30% formalin solution is a solution that contains an equivalent amount of formaldehyde as a solution containing 3 parts by volume formalin (as defined above) to 7 parts by volume solvent.

### II. Introduction

Fixation preserves a cellular sample for subsequent examination. Chemical fixation involves immersing the sample in a volume of chemical fixative. The fixative diffuses through the tissue sample and preserves structures (both chemically and structurally) as close to that of living cells as possible. Cross-linking fixatives, typically aldehydes, create covalent chemical bonds between endogenous biological molecules, such as proteins and nucleic acids, present in the sample. Formaldehyde is the most commonly used fixative in histology. Formaldehyde may be used in various concentrations for fixation, but it primarily is used as 10% neutral buffered formalin (NBF), which is about 3.7% formaldehyde in an aqueous phosphate buffered saline solution. Paraformaldehyde is a polymerized form of formaldehyde, which depolymerizes to provide formalin when heated. Glutaraldehyde operates in similar manner as formaldehyde, but is a larger molecule having a slower rate of diffusion across membranes. Glutaraldehyde fixation provides a more rigid or tightly linked fixed product, causes rapid and irreversible changes, provides good overall cytoplasmic and nuclear detail, but is not ideal for immunohistochemistry staining. Some fixation protocols use a combination of formaldehyde and glutaraldehyde. Glyoxal and acrolein are less commonly used aldehydes. Many other aldehyde-based fixatives are also known.

It is well known that tissue fixation kinetics can be increased by raising the temperature of the fixative. However, placing a tissue sample directly into a heated fixative can cause the outside of the tissue to cross-link well before formalin penetrated to the center of the tissue, which in turn retards or even prevents further diffusion of the fixative into the tissue. As a result, biomolecules in the center of the tissue are heated without any significant cross-linking, rendering these molecules more susceptible to degradation and damage. It is also well-known that extended exposure of samples to fixative solutions can compromise the integrity of the sample and lead to loss of certain biomarkers, particularly labile biomarkers.

It was previously demonstrated that the degree of degradation and damage could be reduced by first pre-soaking the tissue samples in cold fixative to allow the fixative to diffuse throughout the sample, followed by a higher temperature treatment to spur cross-linking. *See* US 2012-0214195 A1. We have unexpectedly found that the cold pre-soaking step can be extended for as long as 14 days without significant loss of tissue.

### III. Samples

In principle, the present methods may be used with any cellular sample type that can be fixed with aldehyde-based fixatives, including tissue samples and cytology samples.

In one embodiment, the sample is a tissue sample. Typically, tissue samples for immersion fixation are limited in size to ensure that fixative diffusion occurs quickly enough and adequately enough to preserve tissue morphology. Thus, certain tissue samples, such as tumor resections and whole organs, must be dissected before fixation to ensure adequate diffusion of the fixative. This is particularly true when the tissue contains analytes of interest that are subject to degradation by residual enzyme activity in the tissue. The present methods, however, increase diffusion speed and thus enable fixation of thicker-than-normal tissue samples. In an embodiment, the tissue may be as large as a tumor resection or a whole organ. In another embodiment, the tissue sample is a tissue biopsy, such as a core needle biopsy.

The present methods and systems are especially useful in fixing clinical samples in which the presence of labile biomarkers (including post-translational modifications to proteins and labile nucleic acids) will be evaluated. In some embodiments, the sample is a clinical tissue sample.

### IV. Fixative Compositions

The present methods are useful with aldehyde-based fixatives. In certain embodiments, the fixative is an aldehyde-based cross-linking fixative, such as glutaraldehyde- and/or formalin-based solutions. Examples of aldehydes frequently used for immersion fixation include:
- formaldehyde (standard working concentration of 5-10% formalin for most tissues, although concentrations as high as 20% formalin have been used for certain tissues);
- glyoxal (standard working concentration 17 to 86 mM);
- glutaraldehyde (standard working concentration of 200 mM).

In one embodiment, the fixative comprises a standard concentration of formaldehyde, glyoxal, or glutaraldehyde. In one exemplary embodiment, the aldehyde-based fixative solution is about 5% to about 20% formalin.

Aldehydes are often used in combination with one another. Standard aldehyde combinations include 10% formalin + 1% (w/v) Glutaraldehyde. Atypical aldehydes have been used in certain specialized fixation applications, including: fumaraldehyde, 12.5% hydroxyadipaldehyde (pH 7.5), 10% crotonaldehyde (pH 7.4), 5% pyruvic aldehyde (pH 5.5), 10% acetaldehyde (pH 7.5), 10% acrolein (pH 7.6), and 5% methacrolein (pH 7.6). Other specific examples of aldehyde-based fixative solutions used for immunohistochemistry are set forth in Table 1:

**Table 1**

| **Solution** | **Standard Composition** |
|---|---|
| Neutral Buffered Formalin | 5-20% formalin + phosphate buffer |
| Formal Calcium | 10% formalin + 10 g/L calcium chloride |
| Formal Saline | 10% formalin + 9 g/L sodium chloride |
| Zinc Formalin | 10% formalin + 1 g/L zinc sulphate |
| Helly's Fixative | 50 mL 100% formalin + 1 L aqueous solution containing 25 g/L potassium dichromate + 10 g/L sodium sulfate + 50 g/L mercuric chloride |
| B-5 Fixative | 2 mL 100% formalin + 20 mL aqueous solution containing 6 g/L mercuric chloride + 12.5 g/L sodium acetate (anhydrous) |
| Hollande's Solution | 100 mL 100% formalin + 15 mL Acetic acid + 1L aqueous solution comprising 25g copper acetate and 40g picric acid |
| Bouin's Solution | 250 mL 100% formalin + 750 mL saturated aqueous picric acid + 50 mL glacial acetic acid |

In certain embodiments, the fixative solution is selected from Table 1.

In the context of concentrations of components of the aldehyde-based fixatives, the term "about" shall be understood to encompass all concentrations outside of the recited range that do not result in a statistically significant difference in diffusion rate in the same type of tissue having the same size and shape as measured by Bauer et al., Dynamic Subnanosecond Time-of-Flight Detection for Ultra-precise Diffusion Monitoring and Optimization of Biomarker Preservation, Proceedings of SPIE, Vol. 9040, 90400B-1 (2014-Mar-20).

Another feature of the methods and systems is that they do not need exogenous degradation inhibitors (such as phosphatase inhibitors, kinase inhibitors, protease inhibitors, or nuclease inhibitors) to substantially preserve labile biomarkers in a state that they can be detected by histochemistry. Therefore, although such degradation inhibitors may be included in the fixative solutions, they are not required. In an embodiment, the aldehyde-based fixative solutions do not contain an effective amount of exogenously added phosphatase inhibitor or kinase inhibitor. In other embodiments, the aldehyde-based fixative solutions do not contain an effective amount of phosphatase inhibitor, kinase inhibitor, protease inhibitor, or nuclease inhibitor.

### V. Fixation Process

Certain disclosed embodiments concern a multi-step, typically a two-step, tissue fixation process for infusing/diffusing a tissue sample using an aldehyde-based fixative solution. During a first processing step, a sample is treated with the aldehyde-based fixative solution under conditions that allow the fixative to diffuse throughout substantially the entire cross-section of the sample. This first step is conducted using a fixative composition for a first period of time, and at a first temperature, that effects substantially complete tissue infusion/diffusion. The second step is to subject the tissue sample to a fixative composition at a second, higher temperature to allow cross-linking to occur. In operation, the first and second processing steps are performed over the course of an extended time period, typically on the order of greater than two days. As shown in the Examples below, the process has been validated up to 14 days, although it likely can be extended for even longer than that.

First, an unfixed tissue sample is immersed in an aldehyde-based fixative solution at a cold temperature. The temperature of the aldehyde-based fixative solution is held at the cold temperature at least long enough to ensure that the fixative has diffused throughout the tissue sample. The minimum amount of time to allow diffusion can be determined empirically using various time and temperature combinations in cold fixatives and evaluating the resulting tissue samples looking at factors, such as preservation of tissue architecture and loss of for preservation of a target analyte by immunohistochemistry (if the analyte is a protein or phosphorylated protein, for example) or *in situ* hybridization (if the target analyte is a nucleic acid, such as miRNA or mRNA). Alternatively, the minimum amount of time of time to allow for diffusion can be determined by monitoring diffusion using, for example, a method as outlined in Bauer et al., Dynamic Subnanosecond Time-of-Flight Detection for Ultra-precise Diffusion Monitoring and Optimization of Biomarker Preservation, Proceedings of SPIE, Vol. 9040, 90400B-1 (2014-Mar-20). An effective temperature range for the first step can include any temperature between the freezing point of the aldehyde-based fixative solution and below 10 °C, for example, about 0 °C to about 7 °C, about 2 °C to about 5 °C, and about 4 °C. In this context, the term "about" shall encompass temperatures that do not result in a statistically significant difference in diffusion rate in the same type of tissue having the same size and shape as measured by Bauer et al., Dynamic Subnanosecond Time-of-Flight Detection for Ultra-precise Diffusion Monitoring and Optimization of Biomarker Preservation, Proceedings of SPIE, Vol. 9040, 90400B-1 (2014-Mar-20). Diffusion of the fixative composition into the tissue sample is continued for a time period effective for diffusion of the composition throughout substantially the entire cross section of the sample.

Once the cold fixative solution has sufficiently diffused throughout the tissue sample, it is stored for an extended period of time either in cold storage (such as a refrigerator or ice bucket) or at ambient temperature (i.e. a temperature from 18 °C to 28 °C) for a cumulative time of greater than two days. In some embodiments, the cumulative time is from greater than two days to up to two weeks or longer, such as from at least 72 hours to 14 days. "Cumulative time" in this context is the sum of the diffusion time and the following cold or ambient temperature extended storage).

If the sample is stored at cold temperature, then it is subjected to a warm temperature treatment (i.e. a temperature of from 18 °C up to 55 °C) for a sufficient amount of time to permit fixation. The temperature associated with the warm temperature treatment typically is ambient or higher, such as higher than about 18 °C. In an embodiment, a temperature range is from ambient up to 50 °C (such as from 20 °C to 50 °C). If the temperature is reaches around 55 °C, however, the sample generally begins to degrade, which may have a deleterious effect on certain subsequent histological reactions. Therefore, temperatures significantly above 50 °C should be avoided for extended periods of time. Thus, in such an embodiment, the upper temperature and second time period should be selected so as to preserve the sample in a state that permits subsequent analyses (such as *in situ* hybridization, histochemical analyses and/or H&E) to proceed effectively. The optimal upper and lower time and temperature limits should be determined empirically based on the particular analysis that will be performed and the sample type being used. In particular, guardbanding of time and temperature ranges should be performed to determine acceptable time/temperature combinations that do not unacceptably compromise tissue architecture and/or analyte detection levels. In some embodiments, the warm temperature treatment is performed in the same fixative solution in which the first processing step is performed. In such an embodiment, the fixative solution may be brought to the second temperature range by active heating (for example, by using a heating element or other heat source) or passive heating (such as by moving the fixative and sample from a cold environment to a warm environment and allowing the temperature of the fixative solution to equilibrate with the environment). In other embodiments, the sample is placed in contact with a fixative solution at a second temperature range by removing the sample from the fixative solution at the first temperature range and immersing the sample in a volume of an aldehyde-based fixative solution at the second temperature range. For example, the fixative solution at the first temperature range could be disposed in a first vessel and the fixative solution at the second temperature range could be disposed in a second vessel, in which case the sample may be physically moved from the first vessel to the second vessel after the first time period has expired. Alternatively, the fixative solution at the first temperature range may be removed from a vessel and replaced with the fixative solution at the second temperature range. As yet another alternative, only a portion of the fixative solution at the first temperature range may be removed, and a hot fixative solution may be added to the remaining fixative solution, such that the resulting combination brings the temperature within the second temperature range. Many other potential arrangements can be envisioned. In any of the embodiments in this paragraph, the fixative solution at the first temperature range may be the same or different from the fixative solution at the second temperature (including differ in the concentration of aldehyde, identity of aldehyde, and/or overall composition).

If the extended storage is at ambient temperature, then additional warm temperature treatment is unnecessary before further tissue processing, although it can be done if desired.

### VI. Further Tissue Processing

As used herein, the phrase "further tissue processing" shall encompass any process following aldehyde fixation that is used to prepare the fixed tissue sample for storage and/or analysis. Many such processes are well-known and would be well understood by a person of ordinary skill in the art. For example, protocols for using zinc formalin, Helly's fixative and Hollande's require a water wash after fixation to remove various contaminates. Some protocols for Bouin's and B-5 suggest storing the fixed samples in 70% ethanol before processing. Additionally, some specimens may be difficult to cut on a microtome because of calcium carbonate or phosphate deposits, and thus may require decalcification. Other post-fixation tissue processing would be well-known to a person having ordinary skill in the art.

In one embodiment, post-fixation tissue processing comprises wax-embedding. In the typical example, the aldehyde-fixed tissue sample is subjected to a series of alcohol immersions to dehydrate the sample, typically using increasing alcohol concentrations ranging from about 70% to about 100%. The alcohol generally is an alkanol, particularly methanol and/or ethanol. After the last alcohol treatment step the sample is then immersed into another organic solvent, commonly referred to as a clearing solution. The clearing solution (1) removes residual alcohol, and (2) renders the sample more hydrophobic for a subsequent waxing step. The clearing solvent typically is an aromatic organic solvent, such as xylene. Wax blocks are formed by applying a wax, typically a paraffin wax, to the sample. Typically, before tissue analysis, the blocks are sliced into thin sections using a microtome. The thin sections may then be mounted on a slide and stored for later analysis and/or subjected to post-processing analysis.

In other examples, the tissue sample may be embedded in resin blocks (such as epoxy or acrylic resins) instead of wax blocks. Exemplary resins include methyl methacrylate, glycol methacrylate, araldite, and epon. Each requires specialized post-fixation processing steps, which are well known in the art.

### VII. Post-processing analysis

Fixed tissue samples obtained by the processes and compositions disclosed herein can be used together with any staining systems and protocol known in the art of histochemistry, as well as affinity histochemistry, immunohistochemistry and *in situ* hybridization. The present invention can also be used together with various automated staining systems, including those marketed by Ventana Medical Systems, Inc. (such as the VENTANA HE600, SYMPHONY, BENCHMARK, and DISCOVERY series automated platforms), Dako (such as the COVERSTAINER, OMNIS, AUTOSTAINER, and ARTISAN series automated slide stainer), and the LEICA ST series stainers. Exemplary systems are disclosed in U.S. Pat. No. 6,352,861, U.S. Pat. No. 5,654,200, U.S. Pat. No. 6,582,962, U.S. Pat. No. 6,296,809, and U.S. Pat. No. 5,595,707.

In an embodiment, specific analytes are detected using immunohistochemistry (IHC). In the typical IHC protocol, a tissue sample is contacted first with an analyte-specific antibody under conditions sufficient to permit specific binding of the analyte-specific antibody to the analyte. In exemplary embodiments, detection of specific analytes is realized through antibodies capable of specific binding to the analyte (or antibody fragments thereof) conjugated with multiple enzymes (e.g. horse radish peroxidase (HRP), alkaline phosphatase (AP). This enzyme-antibody conjugate is referred to as an HRP or AP multimer in light of the multiplicity of enzymes conjugated to each antibody. Multimer technologies are described in U.S. Patent No. 8,686,122. This type of detection chemistry technology is currently marketed by Ventana Medical Systems Inc., as ultra View Universal DAB detection kit (PIN 760-500), ultraView Universal AP Red detection kit (PIN 760-501), ultraView Red ISH DIG detection kit (PIN 760-505), and ultraView SISH DNP detection kit (PIN 760-098). In illustrative embodiments, the approach uses non-endogenous haptens (e.g. not biotin, see U.S. application Ser. No. 12/660,017 for disclosure related to detection chemistries). In illustrative embodiments, a tyramide signal amplification may be used with this approach to further increase the sensitivity and dynamic range of the detection (See PCT/US2011/042849 for disclosure related to detection chemistries).

US Patent US 2012/214195 A1 discloses a method for fixing tissue samples, the method comprising contacting formalin solution and a tissue sample for a first time period and at a temperature of -20°C to 15°C, and then raising the temperature of the tissue sample to a second temperature higher than the first temperature for a second time period.

Any suitable enzyme/enzyme substrate system can be used for the disclosed analysis/detection method. Working embodiments typically used alkaline phosphatase and horseradish peroxidase. If the enzyme is alkaline phosphatase, one suitable substrate is nitro blue tetrazolium chloride/(5-bromo-4-chloro-1H-indol-3-yl)dihydrogen phosphate (NBT/BCIP). If the enzyme is horseradish peroxidase, then one suitable substrate is diaminobenzidine (DAB). Numerous other enzyme-substrate combinations are known to those skilled in the art. For a general review of these, see U.S. Pat. Nos. 4,275,149, and 4,318,980. In some embodiments, the enzyme is a peroxidase, such as horseradish peroxidase or glutathione peroxidase or an oxidoreductase.

U.S. Patent Publication 2008/0102006 describes robotic fluid dispensers that are operated and controlled by microprocessors. U.S. Patent Publication 2011/0311123 describes methods and systems for automated detection of immunohistochemical (IHC) patterns. The automated detection systems disclosed in these patent applications can be used to detect analytes in the fixed tissue samples of the present invention.

In some embodiments, the fixed tissue samples arc analyzed by immunohistochemistry for the presence of post-translationally modified proteins. In the typical process, the fixed tissue sample is contacted with an analyte-binding entity capable of specifically binding to the post-translationally modified protein under conditions sufficient to effect binding of the analyte-binding entity to the post-translationally modified protein; and binding of the analyte-binding entity to the post-translationally modified protein is detected. The precise conditions for effective IHC generally need to be worked on an individual basis, depending upon, for example, the precise antibody used, the type of sample used, sample size, further processing steps, *et cetera.* In an embodiment, the post-translational modification is one that is susceptible to loss during a standard aldehyde fixation process due to residual enzyme activity within the tissue sample. One could determine whether a given post-translational modification is susceptible to residual enzyme activity by treating a sample with an entity that leads to increased presence of the post-translational modification. The sample could then be fixed using a standard technique (such as 24 hour fixation in room temperature NBF) and a fixation process as disclosed herein and the amount of signal detectable in each of the samples can be compared. If signal is absent or significantly lower in the sample fixed according to standard techniques, then one can assume that the post-translational modification is susceptible to degradation by residual enzyme activity. Thus, in an embodiment, the post-translational modification is a post-translational modification that has a lower level of detection in a tissue fixed for 24 hours in room temperature NBF without a cold temperature pre-treatment than in a substantially identical tissue sample that has been fixed using a two-temperature fixation as described above. In an embodiment, the post-translational modification is a diagnostic or prognostic marker for a disease state of the tissue sample. In an embodiment, the post-translational modification is a predictive marker for an effect of a therapy on a disease state of the tissue. In an embodiment, the post-translational modification is a phosphorylation.

In some embodiments, the fixed tissue samples are analyzed by *in situ* hybridization for the presence of specific nucleic acids. In the typical process, the fixed tissue sample is contacted with a nucleic acid probe complementary to the analyte nucleic acid under conditions sufficient to effect specific hybridization of the probe to the analyte nucleic acid; and binding of the nucleic acid probe to the analyte nucleic acid is detected. The precise conditions for effective ISH generally need to be worked on an individual basis, depending upon, for example, the precise nucleic acid probe used, the type of sample used, sample size, further processing steps, *et cetera.* In an embodiment, the analyte nucleic acid is one that is susceptible to loss during a standard aldehyde fixation process due to residual enzyme activity within the tissue sample. One could determine whether a given nucleic acid is susceptible to residual enzyme activity by treating a sample with an entity that leads to increased presence of the nucleic acid. The sample could then be fixed using a standard technique (such as 24 hour fixation in room temperature NBF) and a fixation process as disclosed herein and the amount of signal detectable in each of the samples can be compared. If signal is absent or significantly lower in the sample fixed according to standard techniques, then one can assume that the analyte nucleic acid is susceptible to degradation by residual enzyme activity. Thus, in an embodiment, the analyte nucleic acid has a lower level of detection in a tissue fixed for 24 hours in room temperature NBF without a cold temperature pre-treatment than in a substantially identical tissue sample that has been fixed using a two-temperature fixation as described above. In an embodiment, the analyte nucleic acid is a diagnostic or prognostic marker for a disease state of the tissue sample. In an embodiment, the analyte nucleic acid is a predictive marker for an effect of a therapy on a disease state of the tissue. In an embodiment, the analyte nucleic acid is an RNA molecule, such as mRNA or miRNA.

### EXAMPLES

The following examples are provided to illustrate certain features of working embodiments of the present invention. A person of ordinary skill in the art will appreciate that the scope of the invention is not limited to the features recited in these examples.

### Example 1: Cold temperature guard banding

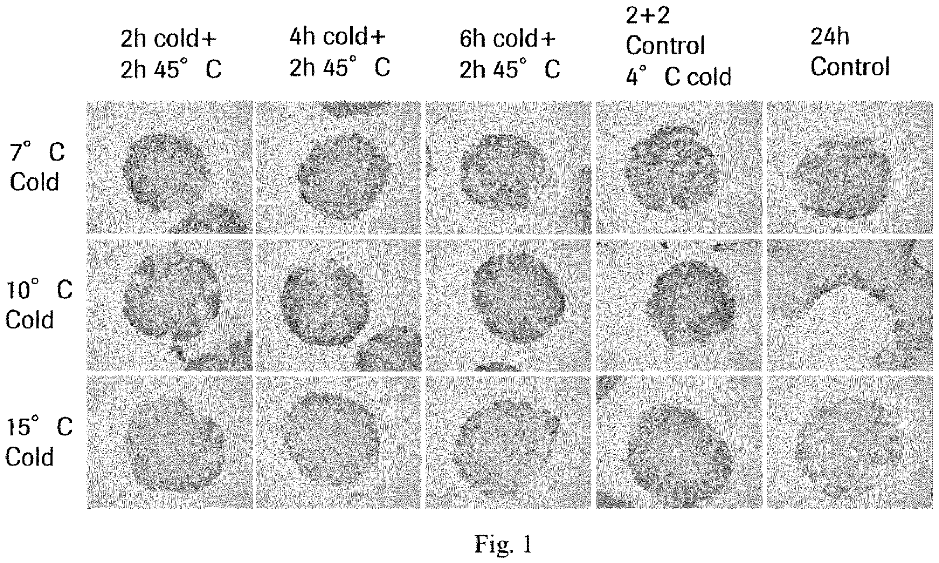

4mm Calu3 Xeongraft tumor cores that were placed into cooled formalin at 7, 10 or 15 °C, respectively, for 2, 4 or 6 hours to form a 9 panel matrix around soak temperature. After the cold soak was completed, tumors were immediately immersed into warm formalin at 45°C for 2 hours. Samples were then processed further in a standard tissue processor set to an overnight cycle. Tissue was sliced in half and embedded cut side down to reveal the edges and middle of the tissue. Control tissues consisted of comparison pieces of the same tumors being fixed with a two-temperature protocol (2 hours 4°C + 2 hours 45°C) and pieces of tumor fixed at RT for 24 hours. Tissues were then stained with anti-pAKT (CST #4060) at a 1:50 dilution on a Ventana DISCOVERY XT automated stainer using the OptiView DAB staining kit (Ventana Medical Systems, Inc.). Results are shown at Fig. 1. As can be seen, there were only small differences between 4 and 7 °C but obvious changes were seen at 10 °C and 15 °C. This suggests that a protocol of 4 °C plus or minus only a few degrees Celsius should give the best results.

### Example 2: Preservation of phosphorylated proteins

Calu3 Xenograft tumors were harvested and placed into the experiment with less than 10 minutes of cold ischemia time. Tumors were cored at 4mm using a disposable biopsy device to ensure all samples were roughly the same size. To test how long samples can sit in cold formalin, pieces of Calu3 tumors (no more than 4mm thick) were placed into 4°C formalin for up to 14 days. After the cold soak was completed, tumors were immediately immersed into warm formalin at 45°C for 2 hours. Samples were then processed further in a standard tissue processor set to an overnight cycle. Tissues were sliced in half and embedded cut side down to reveal the edges and middle of the tissue.

Tissues were stained with anti-pAKT (CST #4060) at a 1:50 dilution on a DISCOVERY XT automated stainer using the OptiView DAB staining kit (Ventana Medical Systems Inc.). This dilution was previously chosen based on a number of similar experiments utilizing Calu3 tumors and this same antibody. To reduce background staining from mouse tissue, staining was performed by substituting a rabbit only form of the linker in the commercial kit.

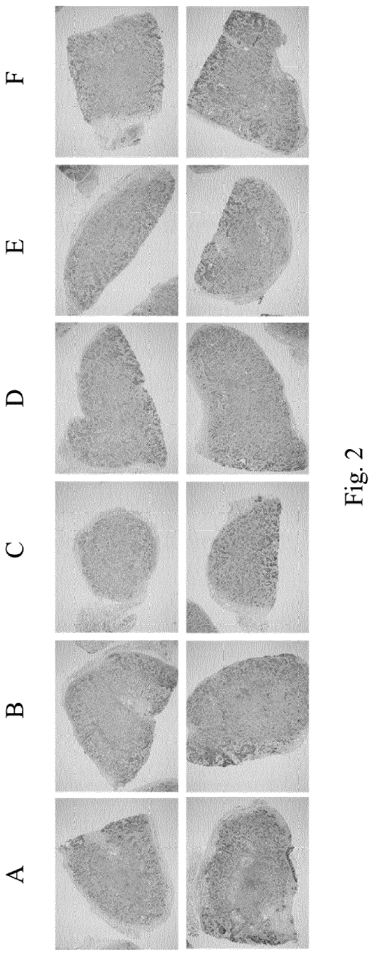

FIG. 2 illustrates the effects of using 4 °C pre-soak processing of Calu3 xenografts over a fourteen day period on phopsho-AKT levels. As can be seen, all samples showed robust staining in samples that had been soaked in 4 °C cold formalin for as long as 14 days. This suggests that tissue can be placed and transported or stored in cold formalin for up to at least 14 days without significant loss of pAKT staining.

### Example 3: Shipping validation

To demonstrate a real-world application of the present fixation process, a shipping study was conducted. A total of 20 Calu-3 xenograft tumors and 20 human tonsil samples were collected. Samples were staggered such that 5 Calu-3 tumors and 5 tonsil samples were shipped in a week. The shipping schedules tested are reproduced below in Table 2:

**Table 2**

| **Shipment Number** | | **Sample Types** | **Length of Shipment** |
|---|---|---|---|
| 1 | | Calu-3 | 6 days |
| | | Tonsil | |
| 2 | | Calu-3 | 52 hours |
| | | Tonsil | |
| 3 | a | Calu3 | 51 hours |
| | b | Tonsil | 117 hours |
| 4 | a | Calu-3 | 28 hours |
| | b | Tonsil | 72 Hours |

Styrofoam-insulated shipping containers were retrofit with data loggers to track the temperature of the package during shipping and frozen inserts to maintain a cold temperature.

### Shipment 1

5 Calu-3 tumors were split into 2 samples each. One half of the tumor was fixed by the 2+2 method as a positive control for controlled fixation. The other half of the tumors were placed into histology cassettes, and the cassettes were labeled and loaded into specimen containers. This procedure was repeated in the afternoon for human tonsil samples that arrive in the afternoon. Specimen containers were placed the data loggers and were placed into a Styrofoam grid which contained a top and bottom for better insulation. Once assembled, the Styrofoam block was placed into either a small or larger shipping container that has frozen inserts. After samples were shipped and received, the tissues were placed into heated formalin for an additional 2 hours, processed overnight into wax blocks and stained for a variety of IHC markers.

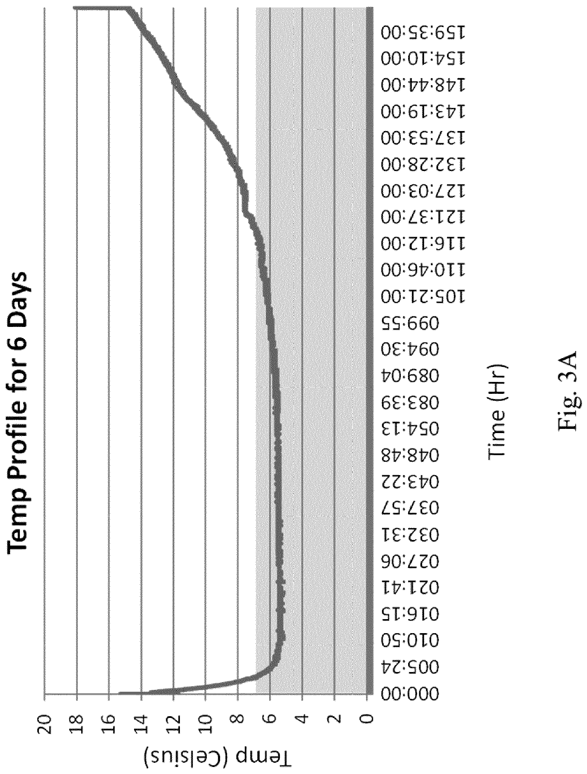

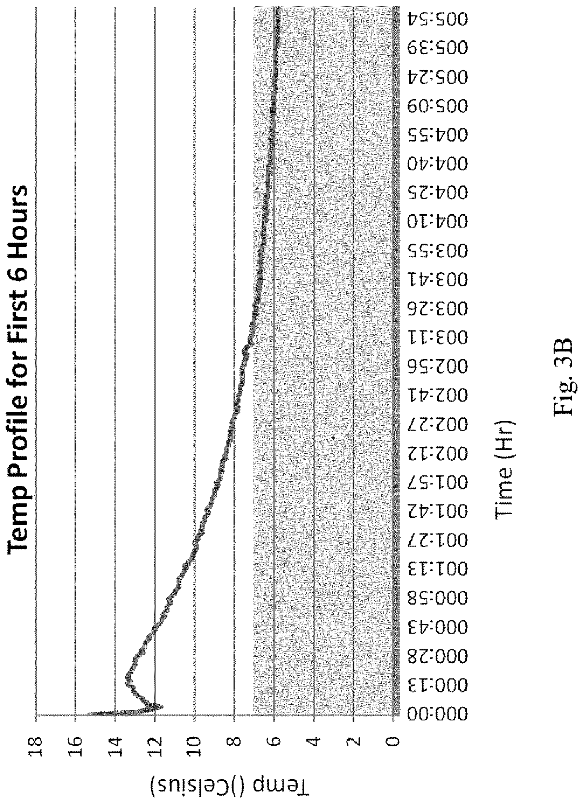

The temperature of the specimen containers during shipping is presented at Figs. 3A & 3B. The temperature spiked to 14 °C after packaging (likely due to the temperature of the data loggers) and slowly cooled to 7 °C in the next 2 ½ hours (right graph). Once cooled to 5 °C, the box maintained temperatures in the safe zone for several days before slowly drifting to 15 °C at which time the samples were removed.

### Shipment 2

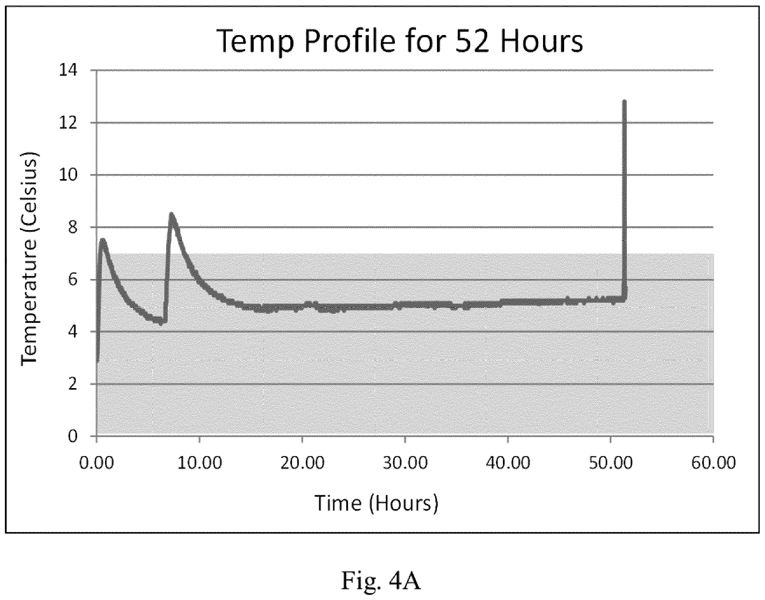

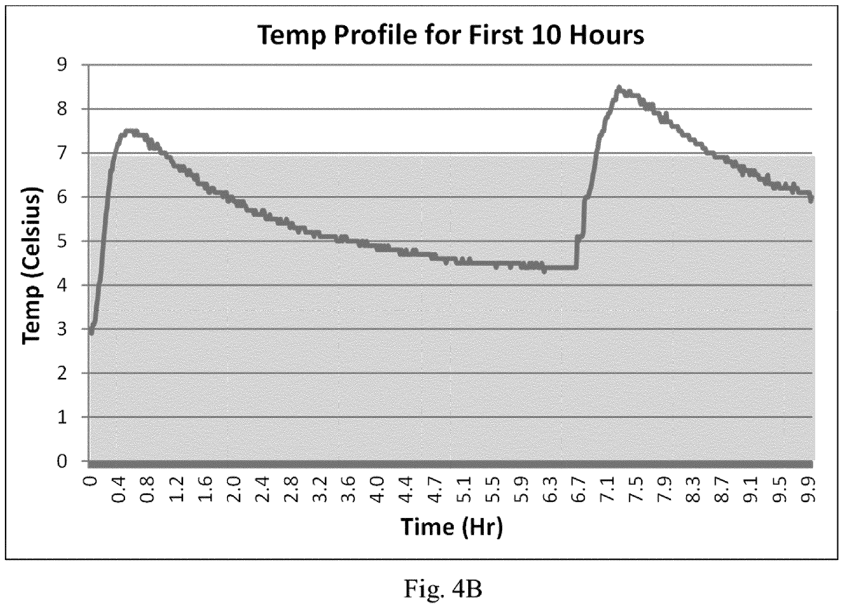

The setup for Shipment 2 was essentially the same as Shipment 1, except that the data loggers were placed in a refrigerator overnight to cool. Samples were harvested in an identical manner to shipment 1 and the data loggers were out of the refrigerator approximately 10 minutes. The temperature of the specimen containers during shipping is presented at Fig. 4. As can be seen from the temperature profiles, two temperature spikes were observed, when the samples were harvested and placed into the shipping container. The first spike corresponds to xenografts harvest and the second spike, several hours later when the tonsil samples were harvested. However, the temperature spikes were just over 7 °C.

### Shipments 3 & 4

Between shipment 2 and 3, the collection procedure was modified slightly to determine if we could maintain the temperature below 7 °C for the entire collection procedure. For this shipment, data loggers were never removed from the refrigerator, only the specimen containers. For example, Calu-3 tumors were received in small batches (2-3 at a time). A corresponding number of specimen containers were placed under a chemical hood and tumors were sectioned, cassettes labeled, clipped into container lids and placed back in the refrigerator within 5 minutes. Specimen containers were placed directly into cooled data loggers and the data loggers were started. When all samples had been processed in this manner, data loggers with corresponding specimen containers were placed into foam packing and placed into a shipping box. The shipping box had been previously conditioned and waiting for the samples. As can be seen, all data loggers registered temperatures below 5.5 °C. Shipment 4 was essentially identical to shipment 3.

### Staining of Shipping Samples

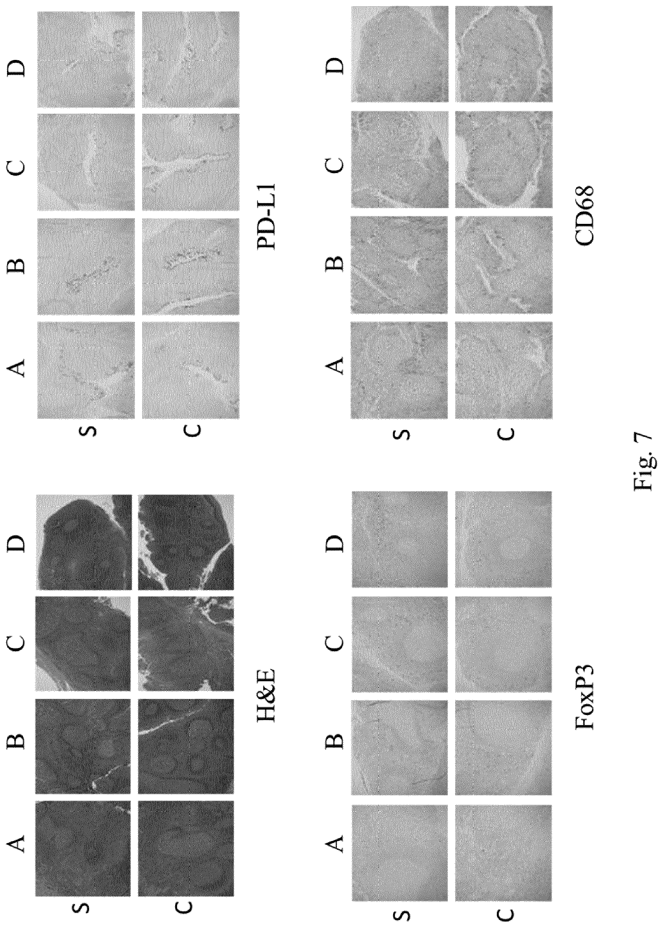

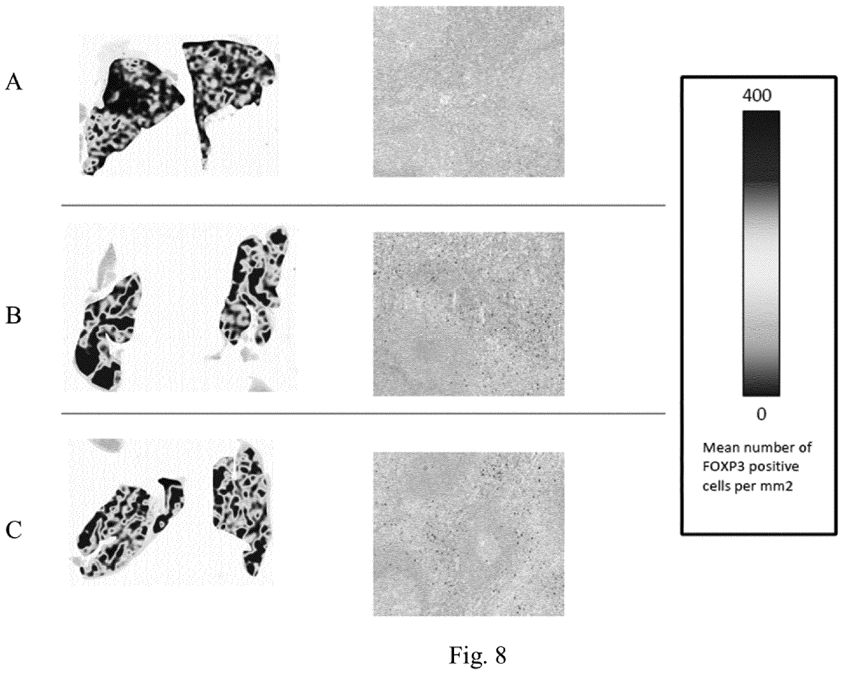

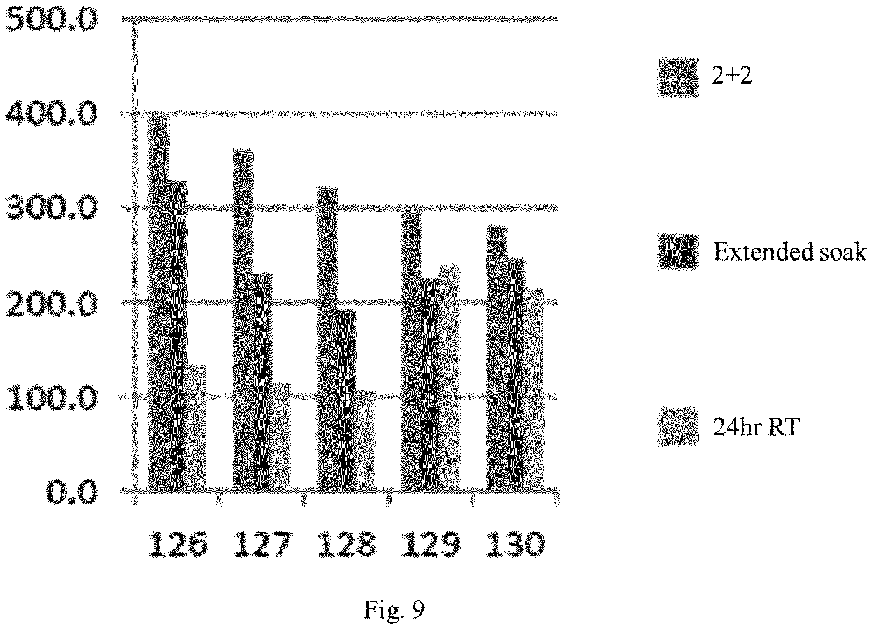

**Human Tonsil** - Human tonsil samples were stained with Hematoxylin and Eosin to determine if there were any tissue morphology issues throughout the shipping process. Samples were compared to control tissues fixed with a 2+2 fixation protocol. All tonsil samples shipped had excellent morphology with no visible defects with any conditions tested (see upper H&E panel). Human tonsil tissues were also stained with PD-L1, FoxP3 and CD68 according to the validation data. All tissues stained identically to control tissues fixed with a 2+2 protocol with all shipping scenarios. FIG. 7 shows representative stains from a subset of the tissues tested. Additionally, when compared to 24 hour fixation, the shipped samples showed significantly better preservation of FoxP3-positive cells. *See* FIGS. 8 and 9.

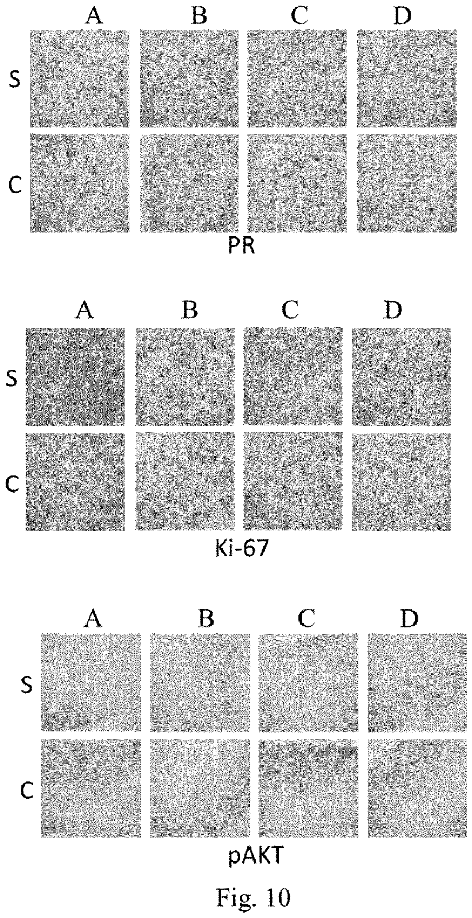

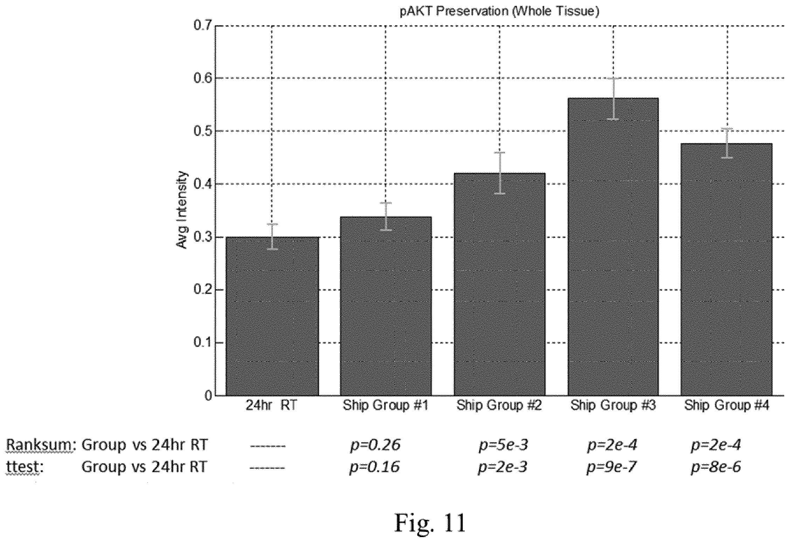

**Calu-3** - Calu-3 samples were stained with PR, Ki-67 and an antibody (CST4060) that recognizes the phosphorylated AKT protein. For total IHC protein staining (PR and Ki-67), results were indistinguishable between control samples fixed with a 2+2 protocol. Robust staining was evident regardless of the shipping conditions, even shipment 1 that had temperatures above the 7 °C zone. It appears that these two proteins are expressed to high levels in the Calu-3 cell model and are stable to slightly elevated temperatures. A different result was obtained when we stained for pAKT. Levels of this labile epitope varied depending on the shipment and temperature conditions compared to controls with a 2+2 fixation protocol. Shipment 1 had initial temperatures up to 14 °C, which led to variable staining between the shipped samples and the 2+2 controls. Variable but better consistency was observed with shipment 2 which had temperatures that just peaked above 7 °C. Better staining consistency was observed with shipments 3 and 4 with almost identical staining compared to the control. FIG. 10 shows representative stains from a subset of the tissues tested. Figure 11 is a bar graph demonstrating the difference in staining intensity between the various shipping samples and the 24 hour room temperature fixation control.

### Example 4: Extended warm soak

Calu3 xenografts were fixed in 10% NBF under a variety of conditions as set forth in Table 3 and evaluated for morphology by H&E stain. "Hot" in table 3 denotes 45 °C for 1 hour. "Cold" indicates 4 °C. Samples were scored on a +, ++, or +++ scale, where + is poor morphology and +++ is the best morphology.

**Table 3**

| **Experiment** | | **Results** (+, ++, +++) | |
|---|---|---|---|
| | | ***Staining level*** | ***Morphology*** |
| **1.1:** | **48 hours cold, 2 weeks RT, hot** | ++ | ++ |
| **1.2:** | **48 hours cold, 2 weeks 37°C, hot** | + | ++ |
| **2.1:** | **1 hour cold, 48 hours RT, hot** | +++ | +++ |
| **2.2:** | **2 hours cold, 48 hours RT, hot** | +++ | +++ |
| **2.3:** | **6 hours cold, 48 hours RT, hot** | +++ | ++ |
| **2.4:** | **6 hours cold, 48 hours 37°C, hot** | + | + |
| **3.1:** | **48 hours RT, hot** | ++ | ++ |
| **4.1:** | **2 hours cold, 4 hours RT, 48 hours cold, hot** | +++ | ++ |
| **5.1:** | **2 hours RT, 48 hours cold, hot** | ++ | ++ |
| **6.1:** | **48 hours cold, hot** | + | + |

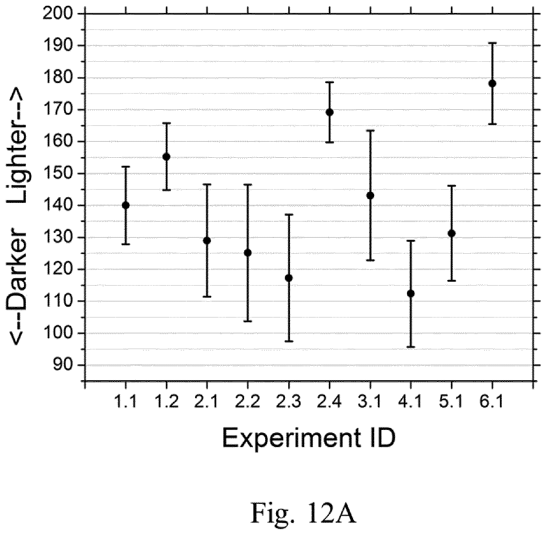

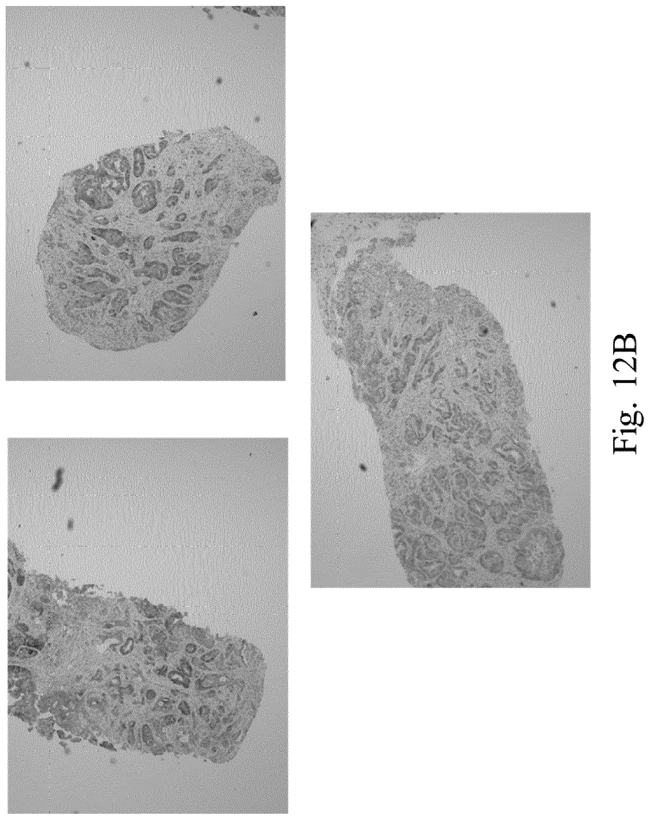

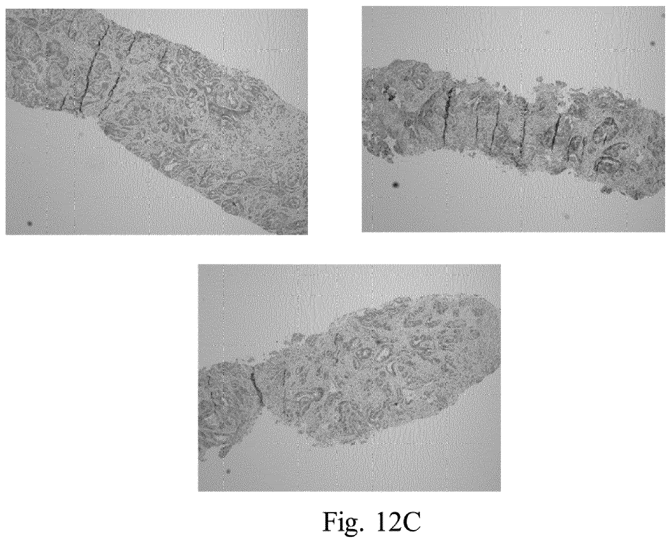

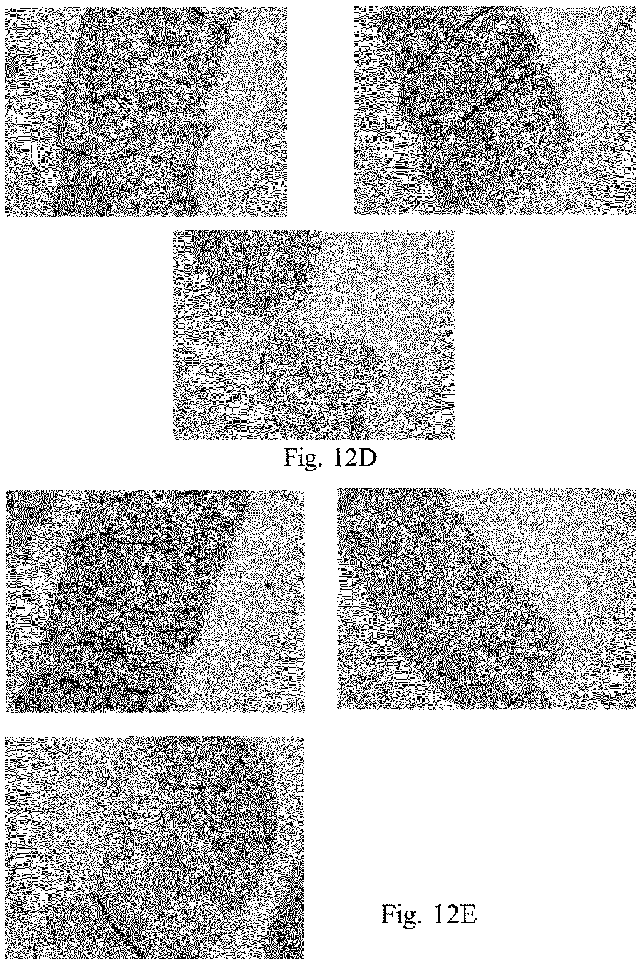

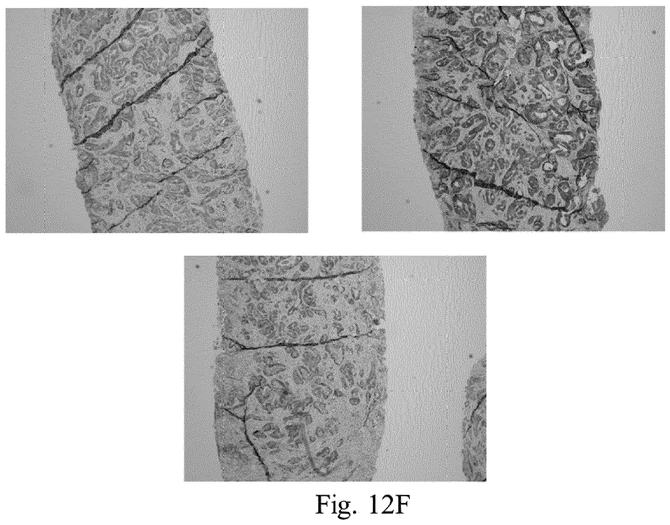

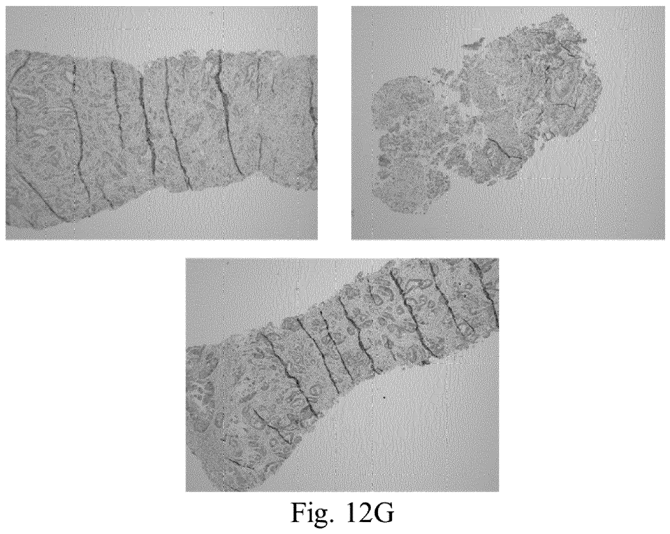

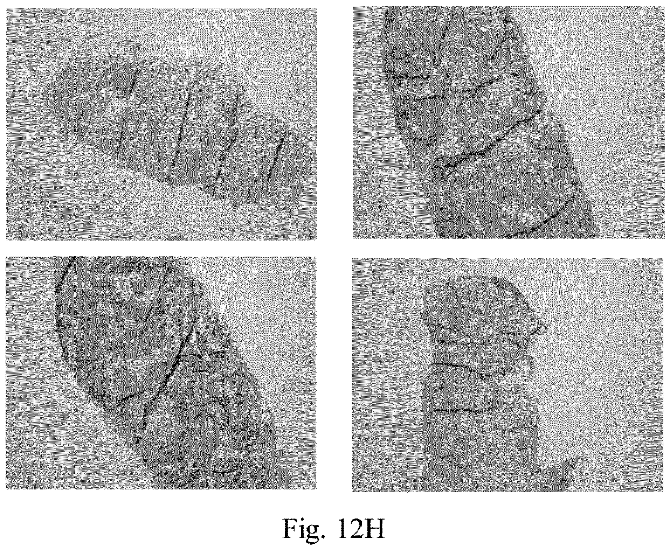

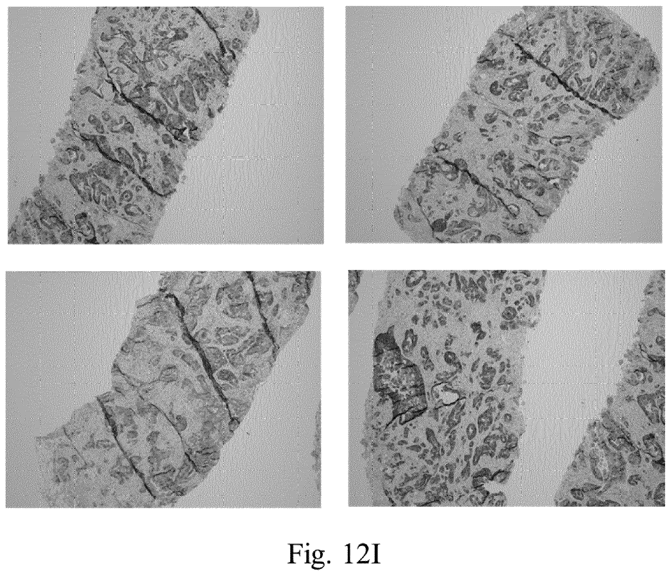

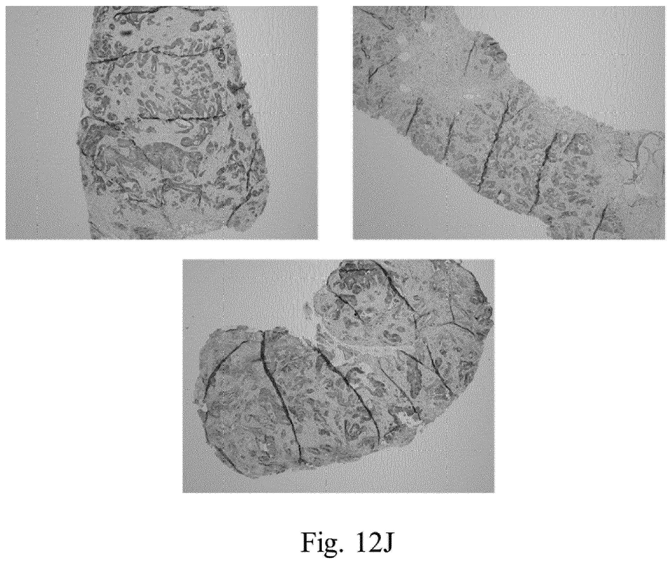

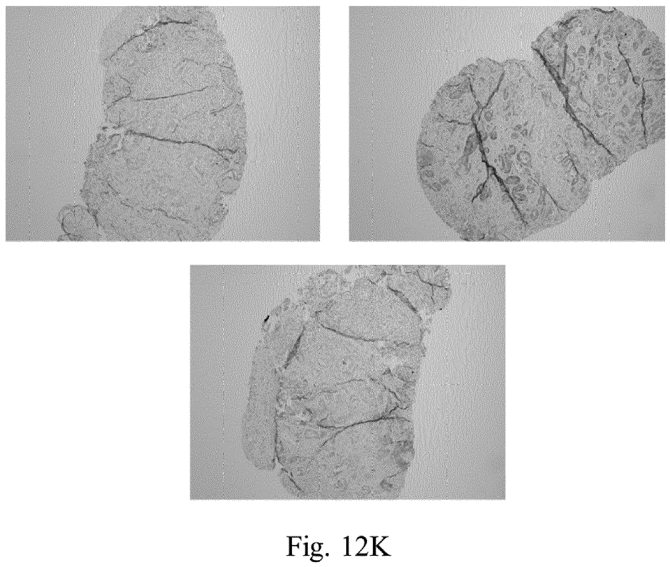

Additionally, the samples were immunohistochemically stained for pAkt. Results are shown at Figs. 12A-12K. These results demonstrate that even a short cold soak enables extended **room temperature** storage without unacceptable loss of morphology or labile markers.

### Example 5: Preservation of nucleic acids (prophetic)

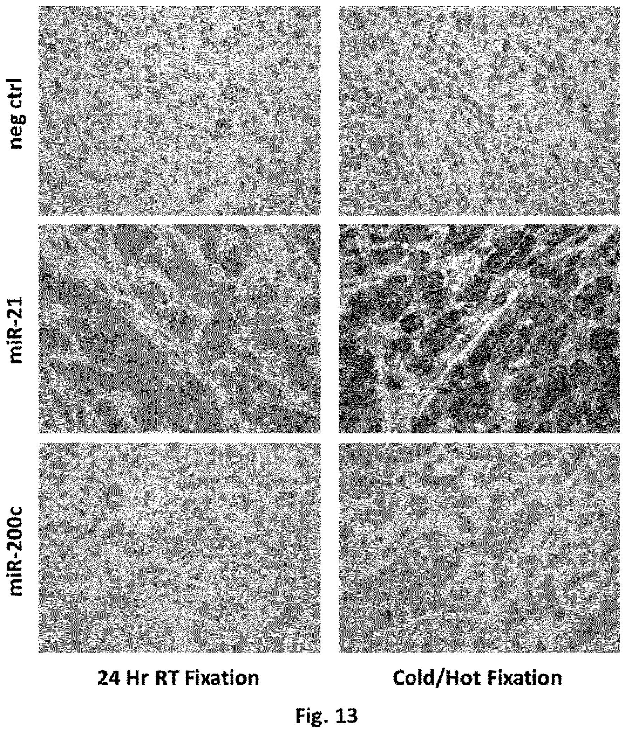

It has previously been demonstrated that nucleic acids (such as mRNA and miRNA) can be sensitive to standard 24 hour room temperature fixation. *See, e.g.,* US 2012-0214195. To illustrate this, the preservation of two miRNA -miR-21 and miR-200c - was evaluated using standard 24 hour room temperature fixation and cold soak followed by 1 hour fixation at 45 °C. 4mm thick pieces of the same human tonsil organ were placed into either room temperature (21-24 °C) 10% neutral buffered formalin for 24 hours or else 2 hours in 4°C formalin followed by 1 hour in 45 °C formalin (Cold/Hot). Tonsil samples were probed for the expression of miR-21 or miR-200c with specific DNA probe sequences to each target. After application of the probe sequence, detection of the bound probe occurred on a VENTANA DISCOVERY XT automated stainer with a silver detection kit. Cold/Hot fixation resulted in an increase in the amount of specific signal in the samples indicating a greater preservation of the miRNA species. Results are shown at FIG. 13. These results indicate that preservation of RNA molecules (such as mRNA and miRNA) can be improved by first exposing the tissue sample to a cold fixative solution for a sufficient amount of time to allow the fixative solution to diffuse into the tissue sample. It is therefore proposed to use a fixation protocol as outlined above to preserve tissue samples for which nucleic acid analysis is desired. A prophetic example for doing so is provided below.

The tissue sample is immersed in an aldehyde-based fixative solution at a cold temperature (e.g., above the freezing point of the fixative solution but less than 10 °C, including for example in a range of from 2 to 7 °C, 2 to 5 °C, or about 4 °C). The temperature of the aldehyde-based fixative solution is held at the cold temperature at least long enough to ensure that the fixative has diffused throughout the tissue sample. The minimum amount of time to allow diffusion can be determined empirically using various time and temperature combinations in cold fixatives and evaluating the resulting tissue samples for preservation of the target nucleic acid using an *in situ* hybridization procedure. Alternatively, the minimum amount of time of time to allow for diffusion can be determined by monitoring diffusion using, for example, a method as outlined in Bauer et al., Dynamic Subnanosecond Time-of-Flight Detection for Ultra-precise Diffusion Monitoring and Optimization of Biomarker Preservation, Proceedings of SPIE, Vol. 9040, 90400B-1 (2014-Mar-20).

Once the cold fixative solution has sufficiently diffused throughout the tissue sample, it is stored for an extended period of time either in cold storage (such as a refrigerator or ice bucket) or at ambient temperature (i.e. a temperature from 18 °C to 28 °C) for a cumulative time of at least 72 hours. "Cumulative time" in this context is the sum of the diffusion time and the following cold or ambient temperature extended storage). If the sample is stored at cold temperature, then it is subjected to a warm temperature treatment (i.e. a temperature of from 18 °C up to 55 °C) for a sufficient amount of time to permit fixation. If the extended storage is at ambient temperature, then additional warm temperature treatment is unnecessary.

After the extended storage period, the tissue sample is subjected to post-fixation processing to prepare it for *in situ* hybridization to detect the target nucleic acid. The tissue sample is washed (if the fixative used requires a wash step), subjected to alcohol dehydration, a clearing solution, and then embedded in paraffin according to standard techniques. The embedded tissue is then sectioned on a microtome, mounted on a slide, and stained for a target messenger RNA (mRNA), microRNA (miRNA), or DNA molecule using an *in situ* hybridization technique, for example, using an automated IHC/ISH slide stainer, such as the VENTANA BENCHMARK or the VENTANA DISCOVERY automated stainer.

## Claims

1. A tissue fixation method comprising:
(a) placing a tissue sample in contact with an aldehyde-based fixative solution in a first temperature range for a first time period, wherein said first temperature range is from above freezing point of the aldehyde-based fixative solution to less than 10 °C, and wherein said first time period is from 72 hours to 14 days; and
(b) after the first time period, placing the tissue sample in contact with an aldehyde-based fixative solution at a temperature in a second temperature range of about 20 °C to less than 55 °C for a second time period, wherein the second time period is from 15 minutes to 4 hours.

2. The method of claim 1, wherein (a) and (b) are completed before further tissue processing is performed.

3. The method of claim 1, wherein the tissue fixation method consists of (a) and (b).

4. The method of any of claims 1-3, wherein the first temperature range is from about 0 °C to about 7 °C, or wherein the first temperature range is from about 2 °C to about 5 °C, or wherein the first temperature range is about 4 °C.

5. The method of any of claims 1-4, wherein the second temperature range is from 20 °C to 50 °C, or wherein the second temperature range is from 35 °C to 45 °C.

6. The method of any of claims 1-5, wherein the second time period is from 15 minutes to 3 hours.

7. The method of any of claims 1-6, wherein the aldehyde-based fixative solution includes a lower alkyl aldehyde, optionally wherein the lower alkyl aldehyde is formaldehyde, glutaraldehyde, glyoxal, or a combination thereof.

8. The method of claim 7, wherein the aldehyde-based fixative solution comprises formalin or is about 10% neutral buffered formalin.

9. The method of any of claims 1-8, wherein the aldehyde-based fixative solution does not contain an effective amount of phosphatase inhibitor, kinase inhibitor, protease inhibitor, or nuclease inhibitor.

10. A fixed tissue sample obtained by the method of any of claims 1 to 9.

11. A histochemical method for staining a tissue sample, said method comprising contacting the fixed tissue sample according to claim 10 with an analyte-binding entity in a manner that causes the analyte-binding entity to bind to an analyte and deposition of a detectable marker onto the fixed tissue sample in close proximity to the analyte to which the analyte-binding entity is bound.

12. The method of claim 11, wherein the analyte comprises a peptide and the analyte-binding entity is an antibody that specifically binds to the analyte, an antibody fragment that specifically binds to the analyte, or a engineered specific binding structures that specifically binds to the analyte.

13. The method of claim 11, wherein the analyte is a protein containing a post-translational modification and the analyte-binding entity does not bind to a protein that lacks the post-translational modification, optionally wherein the post-translational modification is a phosphorylated protein.

14. The method of claim 11, wherein the analyte comprises a nucleic acid and the analyte-binding entity is a nucleic acid probe complementary to a nucleic acid sequence of the analyte.

15. The method of any of claims 11-14, wherein the fixed tissue sample is contacted with the analyte-binding entity on an automated staining platform.

16. A histochemically-stained fixed tissue sample obtained according to a method of any of claims 11-15.

17. A method of detecting an analyte in a tissue sample, said method comprising:
obtaining the histochemically-stained fixed tissue sample of claim 16; and
detecting the presence the detectable label deposited on the histochemically-stained fixed tissue sample.

## Patentansprüche

1. Gewebefixierungsverfahren, umfassend:
(a) Platzieren einer Gewebeprobe in Kontakt mit einer aldehydbasierten Fixierlösung in einem ersten Temperaturbereich für einen ersten Zeitraum, wobei der erste Temperaturbereich von über dem Gefrierpunkt der aldehydbasierten Fixierlösung bis weniger als 10 °C reicht und wobei der erste Zeitraum 72 Stunden bis 14 Tage beträgt; und
(b) nach dem ersten Zeitraum Platzieren der Gewebeprobe in Kontakt mit der aldehydbasierten Fixierlösung bei einer Temperatur in einem zweiten Temperaturbereich von etwa 20 °C bis weniger als 55 °C für einen zweiten Zeitraum, wobei der zweite Zeitraum 15 Minuten bis 4 Stunden beträgt.

2. Verfahren nach Anspruch 1, wobei (a) und (b) beendet werden, bevor weitere Gewebeverarbeitung durchgeführt wird.

3. Verfahren nach Anspruch 1, wobei das Gewebefixierungsverfahren aus (a) und (b) besteht.

4. Verfahren nach einem der Ansprüche 1-3, wobei der erste Temperaturbereich von etwa 0 °C bis etwa 7 °C reicht oder wobei der erste Temperaturbereich von etwa 2 °C bis etwa 5 °C reicht oder wobei der erste Temperaturbereich etwa 4 °C beträgt.

5. Verfahren nach einem der Ansprüche 1-4, wobei der zweite Temperaturbereich von 20 °C bis 50 °C reicht oder wobei der zweite Temperaturbereich von 35 °C bis 45 °C reicht.

6. Verfahren nach einem der Ansprüche 1-5, wobei der zweite Zeitraum 15 Minuten bis 3 Stunden beträgt.

7. Verfahren nach einem der Ansprüche 1-6, wobei die aldehydbasierte Fixierlösung einen Niederalkylaldehyd einschließt, gegebenenfalls wobei der Niederalkylaldehyd Formaldehyd, Glutaraldehyd, Glyoxal oder eine Kombination davon ist.

8. Verfahren nach Anspruch 7, wobei die aldehydbasierte Fixierlösung Formalin umfasst oder etwa 10%iges neutrales gepuffertes Formalin ist.

9. Verfahren nach einem der Ansprüche 1-8, wobei die aldehydbasierte Fixierlösung keine wirksame Menge Phosphataseinhibitor, Kinaseinhibitor, Proteaseinhibitor oder Nukleaseinhibitor enthält.

10. Fixierte Gewebeprobe, erhalten mit dem Verfahren nach einem der Ansprüche 1 bis 9.

11. Histochemisches Verfahren zum Färben einer Gewebeprobe, wobei das Verfahren das Inkontaktbringen der fixierten Gewebeprobe nach Anspruch 10 mit einer analytbindenden Einheit in einer Weise, die das Binden der analytbindenden Einheit an einen Analyten und die Abscheidung eines nachweisbaren Markers auf der fixierten Gewebeprobe in unmittelbarer Nähe des Analyten, an den die analytbindende Einheit gebunden ist, bewirkt.

12. Verfahren nach Anspruch 11, wobei der Analyt ein Peptid umfasst und die analytbindende Einheit ein Antikörper, der spezifisch an den Analyten bindet, ein Antikörperfragment, das spezifisch an den Analyten bindet, oder eine gentechnisch veränderte spezifische Bindungsstruktur, die spezifisch an den Analyten bindet, ist.

13. Verfahren nach Anspruch 11, wobei der Analyt ein Protein ist, das eine post-translationale Modifikation enthält, und die analytbindende Einheit nicht an ein Protein bindet, dem die post-translationale Modifikation fehlt, gegebenenfalls wobei die post-translationale Modifikation ein phosphoryliertes Protein ist.

14. Verfahren nach Anspruch 11, wobei der Analyt eine Nukleinsäure umfasst und die analytbindende Einheit eine zu einer Nukleinsäuresequenz des Analyten komplementäre Nukleinsäuresonde ist.

15. Verfahren nach einem der Ansprüche 11-14, wobei die fixierte Gewebeprobe auf einer automatisierten Färbeplattform mit der analytbindenden Einheit in Kontakt gebracht wird.

16. Histochemisch gefärbte fixierte Gewebeprobe, erhalten gemäß einem Verfahren nach einem der Ansprüche 11-15.

17. Verfahren zum Nachweisen eines Analyten in einer Gewebeprobe, wobei das Verfahren Folgendes umfasst:
Erhalten der histochemisch gefärbten fixierten Gewebeprobe nach Anspruch 16; und
Nachweisen der Gegenwart der nachweisbaren Markierung, die auf der histochemisch gefärbten fixierten Gewebeprobe abgeschieden wurde.

## Revendications

1. Procédé de fixation de tissus comprenant :
(a) le placement d'un échantillon de tissu en contact avec une solution de fixateur à base d'aldéhyde dans une première plage de températures pendant une première durée, dans lequel ladite première plage de températures est d'au-dessus du point de congélation de la solution de fixateur à base d'aldéhyde à moins de 10 °C, et dans lequel ladite première durée est de 72 heures à 14 jours ; et
(b) après la première durée, le placement de l'échantillon de tissu en contact avec une solution de fixateur à base d'aldéhyde à une température dans une seconde plage de températures d'environ 20 °C à moins de 55 °C pendant une seconde durée, dans lequel la seconde durée est de 15 minutes à 4 heures.

2. Procédé selon la revendication 1, dans lequel (a) et (b) sont terminés avant qu'un traitement de tissu ultérieur ne soit réalisé.

3. Procédé selon la revendication 1, dans lequel le procédé de fixation de tissu consiste en (a) et (b).

4. Procédé selon l'une quelconque des revendications 1 à 3, dans lequel la première plage de températures est d'environ 0 °C à environ 7 °C, ou dans lequel la première plage de températures est d'environ 2 °C à environ 5 °C, ou dans lequel la première plage de températures est d'environ 4 °C.

5. Procédé selon l'une quelconque des revendications 1 à 4, dans lequel la seconde plage de températures est de 20 °C à 50 °C, ou dans lequel la seconde plage de températures est de 35 °C à 45 °C.

6. Procédé selon l'une quelconque des revendications 1 à 5, dans lequel la seconde durée est de 15 minutes à 3 heures.

7. Procédé selon l'une quelconque des revendications 1 à 6, dans lequel la solution de fixateur à base d'aldéhyde inclut un alkylaldéhyde court, éventuellement dans lequel l'alkylaldéhyde court est le formaldéhyde, le glutaraldéhyde, le glyoxal ou une combinaison de ceux-ci.

8. Procédé selon la revendication 7, dans lequel la solution de fixateur à base d'aldéhyde comprend du formol ou est du formol neutre tamponné à environ 10 %.

9. Procédé selon l'une quelconque des revendications 1 à 8, dans lequel la solution de fixateur à base d'aldéhyde ne contient pas une quantité efficace d'inhibiteur de phosphatase, d'inhibiteur de kinase, d'inhibiteur de protéase ou d'inhibiteur de nucléase.

10. Échantillon de tissu fixé obtenu par le procédé selon l'une quelconque des revendications 1 à 9.

11. Procédé histochimique de coloration d'un échantillon de tissu, ledit procédé comprenant la mise en contact de l'échantillon de tissu fixé selon la revendication 10 avec une entité se liant à un analyte d'une manière qui amène l'entité se liant à un analyte à se lier à un analyte et le dépôt d'un marqueur détectable sur l'échantillon de tissu fixé à proximité immédiate de l'analyte auquel l'entité se liant à un analyte est liée.

12. Procédé selon la revendication 11, dans lequel l'analyte comprend un peptide et l'entité se liant à un analyte est un anticorps qui se lie spécifiquement à l'analyte, un fragment d'anticorps qui se lie spécifiquement à l'analyte ou une structure de liaison spécifique modifiée qui se lie spécifiquement à l'analyte.

13. Procédé selon la revendication 11, dans lequel l'analyte est une protéine contenant une modification post-traductionnelle et l'entité se liant à un analyte ne se lie pas à une protéine qui ne présente pas la modification post-traductionnelle, éventuellement dans lequel la modification post-traductionnelle est une protéine phosphorylée.

14. Procédé selon la revendication 11, dans lequel l'analyte comprend un acide nucléique et l'entité se liant à un analyte est une sonde d'acide nucléique complémentaire d'une séquence d'acide nucléique de l'analyte.

15. Procédé selon l'une quelconque des revendications 11 à 14, dans lequel l'échantillon de tissu fixé est mis en contact avec l'entité se liant à un analyte sur une plateforme de coloration automatisée.

16. Échantillon de tissu fixé coloré de manière histochimique obtenu selon un procédé selon l'une quelconque des revendications 11 à 15.

17. Procédé de détection d'un analyte dans un échantillon de tissu, ledit procédé comprenant : l'obtention de l'échantillon de tissu fixé coloré de manière histochimique selon la revendication 16 ; et la détection de la présence du marqueur détectable déposé sur l'échantillon de tissu fixé coloré de manière histochimique.
